(19) Europäisches Patentamt
European Patent Office
Office européen des brevets

(11) Veröffentlichungsnummer: **0 523 493 A1**

(12) **EUROPÄISCHE PATENTANMELDUNG**

(21) Anmeldenummer: **92111406.2**

(22) Anmeldetag: **06.07.92**

(51) Int. Cl.5: **C07D 339/08**, C07D 409/14, C07D 409/12, C07D 409/04, A61K 31/385

(30) Priorität: **18.07.91 CH 2144/91**

(43) Veröffentlichungstag der Anmeldung: **20.01.93 Patentblatt 93/03**

(84) Benannte Vertragsstaaten: **AT BE CH DE DK ES FR GB GR IT LI LU MC NL PT SE**

(71) Anmelder: **F. HOFFMANN-LA ROCHE AG**
**Postfach 3255**
**CH-4002 Basel(CH)**

(72) Erfinder: **Eliason, James F.**
**237 Yamate-cho, Naka-ku**
**Yokohama, Kanagawa-ken(JP)**
Erfinder: **Ramuz, Henri**
**Rheinparkstrasse 3**
**CH-4127 Birsfelden(CH)**
Erfinder: **Kaufmann-Schmid, Franz Alois**

**verstorben(DE)**

(74) Vertreter: **Kellenberger, Marcus, Dr. et al**
**Grenzacherstrasse 124 Postfach 3255**
**CH-4002 Basel(CH)**

(54) **Dithiane.**

(57) Verbindungen der Formel

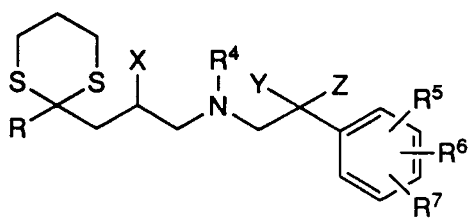

I

worin R einen Rest der Formel

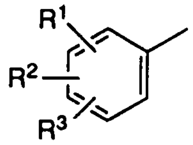

,

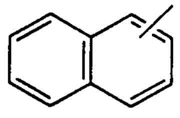

oder

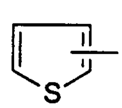

,

(a) (b) (c)

und $R^1$, $R^2$, $R^3$, $R^4$, $R^5$, $R^6$, $R^7$, X, Y und Z die in Anspruch 1 angegebene Bedeutung besitzen mit Ausnahme von rac-N-(3,4-Dimethoxyphenethyl)-2-(3,4-dimethoxyphenyl)-$\beta$,N-dimethyl-m-dithian-2-propylamin sowie deren

EP 0 523 493 A1

Säureadditionssalze besitzen hervorragende Eigenschschaften gegen mehrfachresistente Zellen von malignen Tumoren sowie die Chloroquinresistenz und eignen sich demnach zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria.

Die vorliegende Erfindung betrifft schwefelhaltige Verbindungen. Im speziellen betrifft sie Verbindungen der allgemeinen Formel

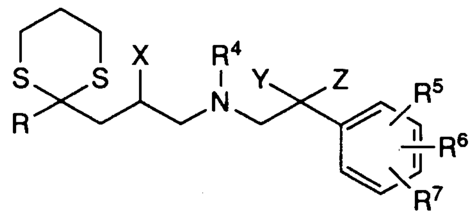

I

worin R einen Rest der Formel

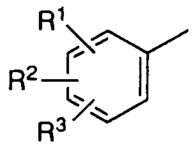

, 

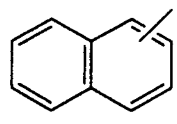

oder

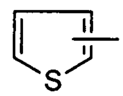

,

(a) (b) (c)

$R^1$, $R^2$ und $R^3$ je Wasserstoff, Halogen, niederes Alkyl, niederes Alkoxy, Aryl-niederes-Alkoxy, niederes Alkylthio, Trifluormethyl oder di-niederes-Alkylamino oder zwei benachbarte dieser Reste zusammen Methylendioxy, Ethylendioxy, Trimethylen oder Tetramethylen; $R^4$ niederes Alkyl; $R^5$, $R^6$ und $R^7$ je Wasserstoff, Halogen, niederes Alkyl, niederes Alkoxy oder Aryl-niederes-Alkoxy oder zwei benachbarte dieser Reste zusammen Methylendioxy oder Ethylendioxy; X Wasserstoff oder niederes Alkyl; Y und Z beide Wasserstoff oder niederes Alkyl oder zusammen Di-, Tri-, Tetra- oder Pentamethylen bedeuten; wobei aber X und Z nicht gleichzeitig Wasserstoff bedeuten und ausserdem rac-N-(3,4-Dimethoxyphenethyl)-2-(3,4-dimethoxyphenyl)-$\beta$,N-dimethyl-m-dithian2-propanamin ausgeschlossen ist;
und Säureadditionssalze davon.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Säureadditionssalze als solche und zur Anwendung als therapeutische Wirkstoffe sowie die Herstellung dieser Verbindungen, ferner diese enthaltende Arzneimittel und die Herstellung solcher Arzneimittel sowie die Verwendung von Verbindungen der Formel I und ihrer pharmazeutisch verwendbaren Säureadditionssalze bei der Bekämpfung bzw. Verhütung von Krankheiten bzw. bei der Verbesserung der Gesundheit, insbesondere zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria. Ebenfalls Gegenstand der vorliegenden Erfindung ist die Verwendung von rac-N-(3,4-Dimethoxyphenethyl)-2-(3,4-dimethoxyphenyl)-$\beta$,N-dimethyl-m-dithian-2-propanamin und dessen pharmazeutisch verwendbaren Säureadditionssalzen zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria bzw. zur Herstellung von entsprechenden Arzneimitteln.

Der in der vorliegenden Beschreibung verwendete Ausdruck "niederes Alkyl" bedeutet geradkettige und verzweigte gesättigte Kohlenwasserstoffreste mit 1-4 Kohlenstoffatomen, d. h. Methyl, Ethyl, n-Propyl, Isopropyl, n-Butyl, Isobutyl, sec.-Butyl und tert.-Butyl. Der Ausdruck "niederes Alkoxy" bedeutet niedere Alkylethergruppen, worin der Ausdruck "niederes Alkyl" die obige Bedeutung hat, d. h. Methoxy, Ethoxy, n-Propoxy, Isopropoxy, n-Butoxy, Isobutoxy, sec.-Butoxy und tert.-Butoxy. Der Ausdruck "Halogen" umfasst die 4 Halogenatome Fluor, Chlor, Brom und Jod. Der Ausdruck "Aryl" bedeutet unsubstituiertes und substituiertes Phenyl, wobei als Substituenten Halogen, niederes Alkyl und niederes Alkoxy zu verstehen sind. Der Ausdruck "austretende Gruppe" bedeutet bekannte Gruppen wie z. B. Halogen, vorzugsweise Chlor oder Brom, und dergleichen.

Bevorzugt sind solche Verbindungen der Formel I, worin $R^4$ Methyl bedeutet. X bedeutet vorzugsweise

Methyl, und das Kohlenstoffatom, an welchem dieses Methyl sitzt, weist die (R,S)-, (R)- oder (S)-Konfiguration auf, besonders bevorzugt die (R)- oder die (S)-Konfiguration. Weiter sind solche Verbindungen der Formel I bevorzugt, worin R einen Rest der Formel (a) oder (b) bedeutet, besonders bevorzugt einen Rest der Formel (a), worin einer der Reste $R^1$, $R^2$ und $R^3$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^1$, $R^2$ und $R^3$ Wasserstoff und der dritte Halogen, insbesondere Chlor, bedeuten. Bevorzugt sind auch solche Verbindungen der Formel I, worin einer der Reste $R^5$, $R^6$ und $R^7$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^5$, $R^6$ und $R^7$ Wasserstoff und der dritte Halogen, insbesondere Chlor, niederes Alkyl, insbesondere Methyl, oder niederes Alkoxy, insbesondere Methoxy, bedeuten. Y und Z bedeuten beide vorzugsweise Wasserstoff oder zusammen Di-, Tri-, Tetra- oder Pentamethylen.

Aus dem obigen folgt, dass solche Verbindungen der Formel I ganz besonders bevorzugt sind, welche durch die folgende Formel dargestellt werden können:

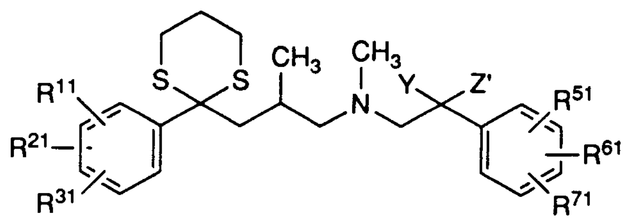

Ia

worin einer der Reste $R^{11}$ , $R^{21}$ und $R^{31}$ Wasserstoff und die beiden anderen Methoxy oder - falls sie benachbart sind - zusammen Methylendioxy oder Ethylendioxy bedeuten oder zwei der Reste $R^{11}$, $R^{21}$ und $R^{31}$ Wasserstoff und der dritte Chlor bedeuten, einer der Reste $R^{51}$, $R^{61}$ und $R^{71}$ Wasserstoff und die beiden anderen Methoxy, oder - falls sie benachbart sind - zusammen Methylendioxy oder Ethylendioxy bedeuten oder zwei der Reste $R^{51}$, $R^{61}$ und $R^{71}$ Wasserstoff und der dritte Chlor, Methyl oder Methoxy bedeuten und Y und Z' beide Wasserstoff oder zusammen Di-, Tri-, Tetra- oder Pentamethylen bedeuten.

Speziell bevorzugte Verbindungen der Formel I sind:

N-[[1-(3,4-Dimethoxyphenyl)cyclohexyl]methyl]-2(3,4-dimethoxyphenyl)-N-methyl-m-dithian-2-propanamin,

rac-N-[[1-(3,4-Dimethoxyphenyl)cyclohexyl]methyl]-β,N-dimethyl-2-(3,4,5-trimethoxyphenyl)-m-dithian-2-propanamin,

2-(3,4-Dimethoxyphenyl)-N-[[1-(3,4-dimethoxyphenyl)cyclopentyl]methyl]-N-methyl-m-dithian-2-propanamin,

rac-2-(4-Chlorphenyl)-N-[[1-(4-chlorphenyl)cyclobutyl]methyl]-β,N-dimethyl-m-dithian-2-propanamin,

(S)-(+)-2-(3,4-Dimethoxyphenyl)-N-[[1-(3,4-dimethoxyphenyl)cyclobutyl]methyl]-β,N-dimethyl-m-dithian-2-propanamin,

(R)-(-)-2-(3,4-Dimethoxyphenyl)-N-[[1-(3,4-dimethoxyphenyl)cyclobutyl]methyl]-β,N-dimethyl-m-dithian-2-propanamin,

(R)-(-)-N-(3,4-Dimethoxyphenethyl)-2-(3,4-dimethoxyphenyl)-β,N-dimethyl-m-dithian-2-propanamin,

(S)-(+)-N-(3,4-Dimethoxyphenethyl)-2-(3,4-dimethoxyphenyl)-β,N-dimethyl-m-dithian-2-propanamin,

rac-N-(4-Chlorphenethyl)-2-(4-chlorphenyl)-β,N-dimethyl-m-dithian-2-propanamin und

rac-N-(3,4-Dimethoxyphenethyl)-β,N-dimethyl-2-(2-naphthyl)-m-dithian-2-propanamin.

Das erfindungsgemässe Verfahren zur Herstellung von Verbindungen der Formel I ist dadurch gekennzeichnet, dass man eine Verbindung der allgemeinen Formel

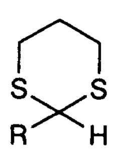

II

worin R die oben angegebene Bedeutung hat,
mit einer Verbindung der allgemeinen Formel

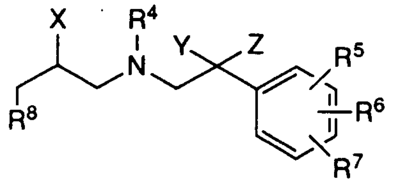

III

worin $R^8$ eine austretende Gruppe bedeutet und X, Y, Z, $R^4$, $R^5$, $R^6$ und $R^7$ die oben angegebene Bedeutung haben,
umsetzt, erwünschtenfalls, eine erhaltene racemische Verbindung der Formel I in die entsprechenden Enantiomeren aufspaltet und/oder erwünschtenfalls eine erhaltene Verbindung der Formel I in ein pharmazeutisch verwendbares Säureadditionssalz überführt.

Die Umsetzung einer Verbindung der Formel II mit einer Verbindung der Formel III kann in an sich bekannter Weise durchgeführt werden. Zweckmässig erfolgt die Reaktion in einem, unter den Reaktionsbedingungen inerten, organischen Lösungsmittel und bei einer Temperatur zwischen etwa -80 °C und einer Temperatur von 40 °C, vorzugsweise zwischen etwa -20 °C und der Raumtemperatur. Als Lösungsmittel kommen Ether, wie abs. Tetrahydrofuran und abs. Diethylether und dergleichen in Frage. Die Reaktion erfolgt in Gegenwart einer starken Base, wie Butyllithium und dergleichen.

Die als Ausgangsmaterial verwendeten Verbindungen der obigen Formel II sind bekannt. Die Verbindungen der obigen Formel III sind teilweise bekannt und teilweise noch neu. So sind diejenigen Verbindungen, worin Y und Z von Wasserstoff verschieden sind und/oder X niederes Alkyl bedeutet, neu. Diese können in an sich bekannter Weise, d. h. in zur Herstellung der bekannten Verbindungen analoger Weise, hergestellt werden.

Die Verbindungen der Formel I können in Säuradditionssalze übergeführt werden, beispielsweise durch Behandlung mit einer anorganischen Säure, wie einer Halogenwasserstoffsäure, beispielsweise Chlorwasserstoff- oder Bromwasserstoffsäure, Schwefelsäure, Phosphorsäure und dergleichen, oder mit einer organischen Säure, wie Oxalsäure, Malonsäure, Bernsteinsäure, Amidosulfonsäure, Fumarsäure, Maleinsäure, Ascorbinsäure, Salicylsäure, Weinsäure, Zitronensäure, Methansulfonsäure, Benzolsulfonsäure, Camphersulfonsäure, Ethandisulfonsäure, Anthrachinon-1,5-disulfonsäure (Journal of Pharmaceutical Sciences, 1977, Vol. 66, Seiten 1-16) und dergleichen.

Von den Säureadditionssalzen der Verbindungen der Formel I sind die pharmazeutisch verwendbaren bevorzugt. Wird im Verlauf des erfindungsgemässen Verfahrens ein Säureadditionssalz einer Verbindung der Formel I erhalten, so kann ein solches Salz in bekannter Weise, z. B. durch Behandlung mit Alkali, in die entsprechende freie Base und diese, erwünschtenfalls, in ein anderes Säureadditionssalz übergeführt werden. Diejenigen Verbindungen der Formel I, welche ein asymmetrisches Kohlenstoffatom enthalten, können in racemischer oder in optisch aktiver Form vorliegen und sowohl die racemischen als auch die optisch aktiven Formen sind Gegenstand der vorliegenden Erfindung.

Man kann diese optisch aktiven Verbindungen erhalten, indem man das Racemat mit einer chiralen Säure umsetzt, das so erhaltene Gemisch der diastereomeren Salze trennt, aus den so erhaltenen Salzen die beiden Basen freisetzt und diese gegebenenfalls mit einer Säure in ihre pharmazeutisch verwendbaren Säureadditionssalze überführt. Da die Verbindungen der Formel I sowie deren Säureadditionssalze im allgemeinen nur sehr schwer kristallisieren, ist dieser Weg im Rahmen der vorliegenden Erfindung nicht bevorzugt. Vielmehr ist die Herstellung dieser optisch aktiven Verbindungen durch Einführung des gewünschten chiralen Zentrums ganz am Anfang der Synthese bevorzugt, und zwar unter Verwendung eines in der Literatur beschriebenen [Helv. Chim. Acta 60, 925-944, (1977)] und in der nachstehenden Formel mit einem Stern markierten chiralen Alkylhalogenids der Formel IV

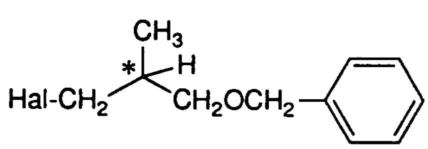

IV

wobei die nächsten Reaktionsstufen zur Herstellung von Verbindungen der allgemeinen Formel

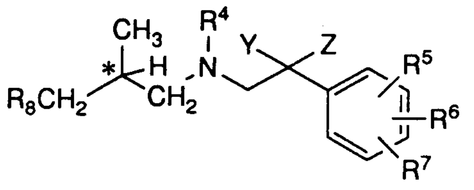

IIIa

worin Y, Z, $R^5$, $R^6$, $R^7$ und $R^8$ die oben angegebene Bedeutung haben, in an sich bekannter Weise durchgeführt werden können.

Der Hauptgrund zum Misserfolg der Behandlung von Krebspatienten ist die Resistenz gegenüber konventionellen Chemotherapeutika. Ein Typ von Arzneimittelresistenz heisst Mehrfachresistenz gegen Cytostatika (Multidrug Resistance); diese Mehrfachresistenz ist durch eine Kreuzresistenz gegenüber funktionell und strukturell nicht verwandten Arzneimittel, wie z. B. Doxorubicin, Vincristin, Vinblastin, Colchicin und Actinomycin D charakterisiert. Das für die Mehrfachresistenz verantwortliche Gen kodiert ein Glycoprotein, Pgp genannt, welches sich als eine energieabhängige Ausflusspumpe für Cytostatika betätigt. Einige Arzneimittel aus verschiedenen therapeutischen und chemischen Klassen besitzen eine gewisse Aktivität zur teilweisen oder vollständigen Behebung der Mehrfachresistenz. Dies ist beschrieben für den Calcium-Kanal Blocker Verapamil [Cancer Res. 41 1967-1972, (1981)], Trifluoperazin, ein Calmodulin-Antagonist [Cancer Res.42, 4730-4733, (1982)], Quinidin, ein Antiarrhythmikum [CancerRes. 44, 4303-4307, (1984)], den Immunsuppressor Cyclosporin A [Br. J. Cancer 54, 235-238, (1986)] und rac-N-(3,4-Dimethoxyphenethyl)-N-methyl-2-(2-naphthyl)-m-dithian-2-propanamin, ein Calcium-Kanal Blocker [Eur. J. Med. Chem. 24, 493-496, (1989)].

Verapamil, Trifluoperazin, Quinidin und Cyclosporin A, welche als spezifische Arzneimittel in der täglichen Therapie eingesetzt werden, sind wegen ihrer pharmakologischen Hauptwirkung als Mittel zur Bekämpfung der Mehrfachresistenz nicht geeignet. rac-N-(3,4-Dimethoxyphenethyl)-N-methyl-2-(2-naphthyl)-m-dithian-2-propanamin, ein relativ toxisches Naphthalinderivat besitzt gegenüber den neuen, hierin beschriebenen Verbindungen der Formel I eine deutlich geringere Wirkung und wurde deshalb in klinischen Versuchen nicht eingesetzt. Es wurde nun gefunden, dass die Verbindungen der Formel I sowie das als Coronardilatator bekannte rac-N-(3,4-Dimethoxyphenethyl)-2-(3,4-dimethoxyphenyl)-$\beta$,N-dimethyl-m-dithian-2-propanamin [US Patentschrift 4.003.914] hervorragende Eigenschaften als Mehrfachresistenz-modifizierende Mittel besitzen, weshalb sie in der Therapie von malignen Tumoren in Kombination mit den üblichen Cytostatika erfolgreich eingesetzt werden können. Die modifizierende Aktivität der Mehrfachresistenz gegenüber Cytostatika von den Verbindungen der Formel I, von rac-N-(3-4-Dimethoxyphenethyl)-2-(3,4-dimethoxyphenyl)-$\beta$,N-dimethyl-m-dithian-2-propanamin sowie von üblichen Referenzsubstanzen wurde wie folgt untersucht:

KB-8-5 Zellen, eine menschliche Zelllinie mit Mehrfachresistenz gegenüber Cytostatika und KB-3-1 Zellen, eine entsprechend empfindliche Zelllinie [Cell. Mol. Genet. 11, 117-126, (1985) und Science 232, 643-645, (1986)] wurden in geeignetem Milieu kultiviert [Cancer Res. 94, 267-275, (1984), Exp. Hematol. 12, 559-567, (1984), Eliason, Ramuz & Kaufmann in Int. J. Cancer 46, 113-117, (1990)].

Der biologische Test basiert auf der Fähigkeit der Testsubstanz, die Konzentration eines Standard-Cytostatikums, welche benötigt wird, um 50 % der Zellen abzutöten, herabzusetzen. Die Prüfung und die Bestimmung der Cytotoxizität beruhen auf der Kapazität von lebenden Zellen, das 3-(4,5-Dimethylthiazol-2-yl)-2,5-diphenyltetrazoliumbromid in das entsprechende blaue Formazan (kolorimetrische Messung) zu reduzieren [J. Immunol. Methods 65, 55-63, (1985)]. In separaten Behältern wurden die Zellen während 2 Tagen inkubiert. Die zur biologischen Untersuchung ausgewählten Verbindungen wurden so zu den Zellkulturen gegeben, dass die nacheinander erhaltenen Konzentrationen $10^{-8}$ M, $10^{-7}$ M, $10^{-6}$ M, $10^{-5}$ M und $10^{-4}$ M erreicht wurden. Vincristin in 4 Verdünnungsreihen ($3 \cdot 10^{-9}$ M, $10^{-8}$ M, $3 \cdot 10^{-8}$ M und $10^{-7}$ M für KB-8-5 Zellen und 30 mal weniger für die KB-3-1 Zellen) wurde dazugegeben. Nach 5 Inkubationstagen wurde die Zellzahl mit Hilfe der Formazanfarbreaktion bei 540 nm gemessen. Zur Beurteilung wurde ein Resistenz-Modifikationsindex, RMI, berechnet, der das Verhältnis zwischen dem $IC_{50}$-Wert von Vincristin in Abwesenheit einer Testsubstanz und dem $IC_{50}$-Wert von Vincristin in Anwesenheit einer Testsubstanz darstellt.

$$RMI = \frac{IC_{50}\ (\text{Kontrollkultur})}{IC_{50}\ (\text{Kultur mit Testsubstanz})}$$

Um sämtliche Aktivitäten von Verbindungen aus verschiedenen Klassen zu vergleichen, wird ein $RMI_{0,1}$ definiert: Darunter versteht man den RMI Wert einer Substanz, der mit der zehnfach niedrigeren Konzentration als der $IC_{50}$-Wert erhalten wird. ($RMI_{0,1} \equiv$ RMI bei 0,1 • $IC_{50}$ der Substanz). In der folgenden Tabelle sind für einige Verbindungen der Formel I sowie für (+) und (-)Verapamil die erhaltenen Resultate zusammengestellt, wobei n die Anzahl der Experimente bedeutet. Angegeben sind auch die präliminären toxikologischen Daten ($DL_{50}$, Maus) nach oraler Gabe der Testverbindung.

| | n | $IC_{50}$ ($\mu$M) | Vincristin $RMI_{0,1}$ | Toxizität mg • $kg^{-1}$ p.o., Maus |
|---|---|---|---|---|
| A | 2 | 14 | 216 | 1250 - 2500 |
| B | 2 | 14 | 159 | 625 - 1250 |
| C | 2 | 21 | 224 | 1000 - 2000 |
| D | 2 | 21 | 163 | 1000 - 2000 |
| E | 2 | 7,7 | 24 | 2500 - 5000 |
| (+) Verapamil | 4 | 62 | 12 | 250 - 500 |
| (-) Verapamil | 4 | 72 | 47 | 60 - 120 |

A = rac-2-(4-Chlorphenyl)-N-(3,4-dimethoxyphenethyl)-β,N-dimethyl-m-dithian-2-propanamin-hydrochlorid,
B = rac-N-(3,4-Dimethoxyphenethyl)-β,N-dimethyl-2-(4-tolyl)-m-dithian-2-propanamin-hydrochlorid,
C = rac-N-[[1-(3,4-Dimethoxyphenyl)cyclobutyl]methyl]-β,N-dimethyl-2-(3,4,5-trimethoxyphenyl)-m-dithian-2--propanamin-hydrochlorid,
D = rac-2-(3,4-Dimethoxyphenyl)-N-[[1-(3,4-dimethoxyphenyl)cyclobutyl]methyl]-β,N-dimethyl-m-dithian-2--propanamin-hydrochlorid,
E = rac-N-(4-Chlorphenethyl)-2-(3,4-dimethoxyphenyl)-β,N-dimethyl-m-dithian-2-propanamin-hydrochlorid.

Die Dithiane der Formel I, das rac-N-(3,4-Dimethoxyphenethyl)-2-(3,4-dimethoxyphenyl)-β,N-dimethyl-m-dithian-2-propanamin und deren Salze besitzen hervorragende Eigenschaften gegen mehrfachresistente Zellen von malignen Tumoren. Diese Verbindungen können in Vereinigung, zusammen oder getrennt, mit einem oder mehreren üblichen Antikrebsmitteln, wie Vinca-Alkaloiden oder Epipodophyllotoxinen (z. B. Vincristin, Vinblastin, Vindesin, Etoposin, Teniposid), Antibiotika (z. B. Adriamycin, Daunorubicin, Bleomycin, Mithramicin), Interchalatoren (z. B. Amonafide), Antimetaboliten (z. B. Fluoruracil), Alkylantien (z. B. Cyclophosphamid, Cisplatin) eingesetzt werden. Die Erfindung betrifft daher auch Erzeugnisse enthaltend eine dieser Verbindungen und ein Cytostatikum als Kombinationspräparat zur gleichzeitigen, getrennten oder zeitlich abgestuften Anwendung in der cytostatischen Therapie. Durch die neuen Kombinationen wird nicht nur die Bildung von Primärtumoren, sondern auch die Bildung von Metastasen verhindert oder stark eingeschränkt. Wie erwähnt, können diese Verbindungen zusammen oder getrennt mit den Cytostatika verabreicht werden. Bevorzugt ist jedoch die getrennte vorherige Applikation dieser Verbindungen, welche durch orale Gabe erfolgt, während die Cytostatika oral oder parenteral gegeben werden. Die Cytostatika können in einer kleineren oder in einer ähnlichen Menge wie in der üblichen Therapie dosiert werden. Die Dosierung dieser Verbindungen hängt vom Alter, Zustand und Gewicht des Patienten sowie von der Applikationsart ab. In der Regel beträgt die Wirkstoffdosis etwa 1 bis 50 mg•$kg^{-1}$ Körpergewicht bei oraler Gabe und etwa 0,1 mg bis 3 mg•$kg^{-1}$ Körpergewicht bei parenteraler Gabe in einer Bolus Injektion oder bei einer langsam durchgeführten intravenösen Infusion. Die angegebenen Dosierungen sind jedoch nur als Beispiele zu verstehen und können je nach der Schwere des zu behandelnden Falles und im Ermessen des behandelnden Arztes abgeändert werden.

Die Verbindungen der Formel I und deren pharmazeutisch verwendbare Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen verabreicht werden. Die Verabreichung kann aber auch rektal, z. B. in Form von Suppositorien, oder parenteral, z. B. in Form von Injektionslösungen, erfolgen. Wie weiter vorne erwähnt, sind Arzneimittel, enthaltend eine Verbindung der Formel I oder ein pharmazeutisch verwendbares Säureadditionssalz davon ebenfalls Gegenstand der vorliegenden Erfindung, weiterhin auch ein Verfahren zur Herstellung derartiger Arzneimittel, welches dadurch gekennzeichnet ist, dass man eine oder mehrere Verbindungen der Formel I und/oder ein pharmazeutisch verwendbares Säureadditionssalz davon und erwünschtenfalls ein oder mehrere therapeutisch inerte, anorganische oder organische Excipientien in eine galenische Darreichungsform bringt.

Zur Herstellung von Tabletten, Lacktabletten, Dragées und Hartgelatinekapseln kann man als Excipientien z. B. Lactose, Maisstärke oder Derivate davon, Talk, Stearinsäure oder deren Salze und dergleichen verwenden.

Für Weichgelatinekapseln eignen sich als Excipientien z. B. vegetabile Oele, Wachse, Fette, halbfeste und flüssige Polyole und dergleichen.

Zur Herstellung von Lösungen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin, vegetabile Oele und dergleichen.

Für Suppositorien eignen sich als Excipientien z. B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemitel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Die folgenden Beispiele sollen die vorliegende Erfindung erläutern, sie jedoch in keiner Weise einschränken. Alle Temperaturen sind in Celsius Graden angegeben.

Beispiel 1

(A) Eine Lösung von 18,6 g (0,0759 Mol) 1-(3,4-Dimethoxyphenyl)-1-cyclohexylcarbonitril [J. Org. Chem. 36, 1308(1971)] in einem Gemisch von 300 ml abs. Methanol und 400 ml flüssigem Ammoniak wurde mit 5,0 g Raney-Cobalt versetzt, und das Gemisch in einem Schüttelautoklaven bei einer Temperatur von 70° und einem Druck von $10^7$ Pa hydriert. Danach wurde das Gemisch auf -20° abgekühlt und filtriert, und die Lösung unter vermindertem Druck eingedampft. Das zurückbleibende Oel wurde bei 160° und einem Druck von 390 Pa destilliert, wobei man 1-(3,4-Dimethoxyphenyl)-1-cyclohexanmethanamin als ein farbloses Oel erhielt, welches direkt in die nächste Stufe eingesetzt wurde.

(B) Eine Lösung von 7,73 g (0,031 Mol) 1-(3,4-Dimethoxyphenyl)-1-cyclohexanmethanamin, 4,3 ml (0,00348 Mol) N-Ethylmorpholin und 0,3 g Dimethylaminopyridin in 100 ml abs. Tetrahydrofuran wurde auf -10° abgekühlt und innerhalb von 10 Minuten mit 4,4 ml (0,0348 Mol) Chlorameisensäureisobutylester versetzt. Das Gemisch wurde dann 18 Stunden bei Raumtemperatur gerührt und filtriert, und die Lösung unter vermindertem Druck eingedampft. Der Rückstand wurde in Essigester gelöst, und die organische Lösung erst mit 1 N Salzsäure und dann mit einer gesättigten Natriumbicarbonat-Lösung gewaschen, über Magnesiumsulfat getrocknet und unter vermindertem Druck eingedampft. Das zurückbleibende Oel wurde mit 100 ml abs. Tetrahydrofuran und 2,35 g (0,062 Mol) Lithiumaluminiumhydrid versetzt, und das Gemisch 2 Stunden zum Rückfluss erhitzt, danach abgekühlt und mit einer gesättigten Natriumsulfat-Lösung versetzt. Nach dem Filtrieren des Gemisches und Eindampfen des Lösungsmittels erhielt man ein Oel, welches bei 153°/260 Pa destilliert wurde, wobei man 1-(3,4-Dimethoxyphenyl)-N-methyl-1-cyclohexanmethanamin als ein farbloses Oel erhielt, welches direkt in die nächste Stufe eingesetzt wurde.

(C) Eine Lösung von 3,40 g (0,0129 Mol) 1-(3,4-Dimethoxyphenyl)-N-methyl-1-cyclohexanmethanamin in 50 ml abs. Dimethylformamid wurde mit 3,57 g (0,0258 Mol) wasserfreiem Kaliumcarbonat versetzt. Das Gemisch wurde bei 5° gerührt und mit 1,4 ml (0,0129 Mol) 1-Brom-3-chlorpropan versetzt und anschliessend zunächst 4 Stunden bei 30° und danach 16 Stunden bei Raum temperatur gerührt. Das Lösungsmittel wurde dann unter vermindertem Druck bei Raumtemperatur eingedampft, und der Rückstand mit Wasser versetzt. Das Gemisch wurde dreimal mit Ether extrahiert, und die organischen Extrakte über Magnesiumsulfat getrocknet, filtriert und unter vermindertem Druck eingedampft. Der ölige

Rückstand wurde bei einem Druck von 300 Pa bei 195° destilliert, wobei N-(3-Chlorpropyl)-1-(3,4-dimethoxyphenyl)-N-methylcyclohexanmethanamin als ein farbloses dickflüssiges Oel erhalten wurde, welches direkt in die nächste Stufe eingesetzt wurde.

(D) In einem Sulfierkolben wurde, unter Begasung mit getrocknetem Argon, eine Lösung von 2,56 g (0,01 Mol) 2-(3,4-Dimethoxyphenyl)-m-dithian in 20 ml abs. Tetrahydrofuran auf -60° abgekühlt und innerhalb von 15 Minuten tropfenweise mit 4,68 ml (0,0075 Mol) einer Lösung von Butyllithium in Hexan versetzt, wobei die Temperatur bei -60° gehalten wurde. Danach wurde die Lösung bei -20° 2 Stunden gerührt, dann wieder auf - 60° abgekühlt und mit einer Lösung von 1,70 g (0,005 Mol) N-(3-Chlorpropyl)-1-(3,4-dimethoxyphenyl)-N-methylcyclohexanmethanamin in 20 ml abs. Tetrahydrofuran tropfenweise versetzt. Nach 16 Stunden bei -20° und 4 Stunden bei Raumtemperatur wurde die Lösung auf Eiswasser gegossen, und das Gemisch dreimal mit Ether gewaschen. Die etherischen Extrakte wurden über Magnesiumsulfat getrocknet, filtriert und unter vermindertem Druck eingedampft. Das zurückbleibende Oel wurde an Kieselgel 60 mit Toluol/Essigester (8:2) chromatographiert. Nach Eindampfen des Lösungsmittelgemisches wurde ein farbloses Oel erhalten, welches in wenig abs. Dioxan gelöst und mit einem Ueberschuss Chlorwasserstoff in abs. Dioxan versetzt wurde, wobei N-[[1-(3,4-Dimethoxyphenyl)-cyclohexyl]methyl]-2(3,4-dimethoxyphenyl)-N-methyl-m-dithian-2-propanamin-hydrochlorid als ein fester weisser Schaum erhalten wurde.

| $C_{31}H_{45}NO_4S_2 \cdot HCl$: | | | |
|---|---|---|---|
| Ber.: | C 62.44, | H 7.78, | N 2.35 % |
| Gef.: | C 62.68, | H 8.16, | N 2.28 %. |

Beispiel 2

In analoger Weise wie in Beispiel 1, Absatz (D) beschrieben, wurde aus 2-(3,4,5-Trimethoxyphenyl)-m-dithian und N-(3-Chlorpropyl)-1-(3,4-dimethoxyphenyl)-N-methylcyclohexanmethanamin das N-[[1-(3,4-Dimethoxyphenyl)cyclohexyl]methyl]-N-methyl-2-(3,4,5-trimethoxyphenyl)-m-dithian-2-propanamin-hydrochlorid als Festkörper erhalten.

| $C_{32}H_{47}NO_5S_2 \cdot HCl$: | | | |
|---|---|---|---|
| Ber.: | C 61.37, | H 7.73, | N 2.24 % |
| Gef.: | C 61.08, | H 8.07, | N 2.14%. |

Beispiel 3

(A) In analoger Weise wie in Beispiel 1, Absatz (C) beschrieben, wurde aus 1-(3,4-Dimethoxyphenyl)-N-methyl-1-cyclohexanmethanamin und 1-Brom-2-methyl-3-chlorpropan das rac-N-(3-Chlor-2-methylpropyl)-1-(3,4-dimethoxyphenyl)-N-methylcyclohexanmethanamin als ein dickflüssiges Oel erhalten, welches direkt in die nächste Stufe eingesetzt wurde.

(B) In analoger Weise wie in Beispiel 1, Absatz (D) beschrieben, wurde aus 2-(3,4-Dimethoxyphenyl)-m-dithian und rac-N-(3-Chlor-2-methylpropyl)-1-(3,4-dimethoxyphenyl)-N-methylcyclohexanmethanamin das rac-N-[[1-(3,4-Dimethoxyphenyl)cyclohexyl]methyl]-2-(3,4-dimethoxyphenyl)-$\beta$,N-dimethyl-m-dithian-2-propanamin als dickes Oel erhalten.

| $C_{32}H_{47}NO_4S_2$: | | | |
|---|---|---|---|
| Ber.: | C 66.98, | H 8.26, | N 2.44 % |
| Gef.: | C 66.89, | H 8.29, | N 2.43 %. |

Beispiel 4

In analoger Weise wie in Beispiel 1, Absatz (D) beschrieben, wurde aus 2-(3,4,5-Trimethoxyphenyl)-m-dithian und rac-N-(3-Chlor-2-methylpropyl)-1-(3,4-dimethoxyphenyl)-N-methylcyclohexanmethanamin das rac-N-[[1-(3,4-Dimethoxyphenyl)cyclohexyl]methyl]-β,N-dimethyl-2-(3,4,5-trimethoxyphenyl)-m-dithian-2-propanamin als dickflüssiges Oel erhalten.

| $C_{33}H_{49}NO_5S_2$: | | | |
|---|---|---|---|
| Ber.:<br>Gef.: | C 65.64,<br>C 65.63, | H 8.18,<br>H 8.21, | N 2.32 %<br>N 2.34 %. |

Beispiel 5

(A) In analoger Weise wie in Beispiel 1, Absatz (A) beschrieben, wurde aus 1-(3,4-Dimethoxyphenyl)-1-cyclopentylcarbonitril [J. Org. Chem. 36, 1308 (1971)], 1-(3,4-Dimethoxyphenyl)-1-cyclopentanmethanamin als ein farbloses Oel erhalten, welches direkt in die nächste Stufe eingesetzt wurde.

(B) Eine Lösung von 13,6 g(0,058 Mol) 1-(3,4-Dimethoxyphenyl)-1-cyclopentanmethanamin in 100 ml abs. Methylenchlorid wurde innerhalb von 15 Minuten mit 8,84 ml (0,0638 Mol) Trifluoressigsäureanhydrid versetzt und 2 Stunden zum Rückfluss erhitzt, worauf das Gemisch abgekühlt und auf 300 ml Eiswasser gegossen wurde. Die organische Phase wurde abgetrennt, über Magnesiumsulfat getrocknet und unter vermindertem Druck eingedampft. Der feste Rückstand wurde in wenig Methylenchlorid gelöst, und die Lösung portionenweise mit Hexan versetzt. So erhielt man N-[[1-(3,4-Dimethoxyphenyl)-cyclopentyl]methyl]-2,2,2-trifluoracetamid als Festkörper vom Schmelzpunkt 109-110°. MS: 331 $(M)^{+\bullet}$.

(C) Eine Lösung von 3,3 g (0,01 Mol) N-[[1-(3,4-Dimethoxyphenyl)cyclopentyl]methyl]-2,2,2-trifluoracetamid in 200 ml abs. Aceton wurde mit 1,5 ml (0,0248 Mol) Methyljodid versetzt. Bei einer Temperatur von 10-12° wurden innerhalb 30 Minuten portionenweise 2,75 g (0,049 Mol) pulverisiertes Kaliumhydroxid zugegeben. Nach 10 Minuten wurde das Gemisch filtriert, und das Filtrat unter vermindertem Druck eingedampft. Der Rückstand wurde in einer Lösung von 1,2 g Kaliumhydroxid in 100 ml Wasser aufgenommen. Nach einstündigem Erhitzen zum Rückfluss wurde die Lösung mit Ether extrahiert. Die organischen Extrakte wurden über Magnesiumsulfat getrocknet und eingedampft. So erhielt man 1-(3,4-Dimethoxyphenyl)-N-methyl-1-cyclopentanmethanamin als ein farbloses Oel (Sdp. 145°/260 Pa), welches direkt in die nächste Stufe eingesetzt wurde.

(D) In analoger Weise wie in Beispiel 1, Absatz (C) beschrieben, wurde aus 1-(3,4-Dimethoxyphenyl)-N-methyl-1-cyclopentanmethanamin und 1-Brom-3-chlorpropan das N-(3-Chlorpropyl)-1-(3,4-dimethoxyphenyl)-N-methylcyclopentanmethanamin als ein dickflüssiges Oel (Sdp. 170°/260 Pa) erhalten, welches direkt in die nächste Stufe eingesetzt wurde.

(E) In analoger Weise wie in Beispiel 1, Absatz (D) beschrieben, wurde aus 2-(3,4-Dimethoxyphenyl)-m-dithian und N-(3-Chlorpropyl)-1-(3,4-dimethoxyphenyl)-N-methylcyclopentanmethanamin das 2-(3,4-Dimethoxyphenyl)-N-[[1-(3,4-dimethoxyphenyl)cyclopentyl]methyl]-N-methyl-m-dithian-2-propanamin-hydrochlorid als Festkörper erhalten.

| $C_{30}H_{43}NO_4S_2 \cdot HCl$: | | | |
|---|---|---|---|
| Ber.:<br>Gef.: | C 61.88,<br>C 61.83, | H 7.62,<br>H 8.11, | N 2.41 %<br>N 2.41 %. |

Beispiel 6

In analoger Weise wie in Beispiel 1, Absatz (D) beschrieben, wurde aus 2-(3,4,5-Trimethoxyphenyl)-m-dithian und N-(3-Chlorpropyl)-1-(3,4-dimethoxyphenyl)-N-methylcyclopentanmethanamin das N-[[1-(3,4-Dimethoxyphenyl)cyclopentyl]methyl]-N-methyl-2-(3,4,5-trimethoxyphenyl)-m-dithian-2-propanamin als dickflüssiges Oel erhalten.

| $C_{31}H_{45}NO_5S_2$: | | | |
|---|---|---|---|
| Ber.: | C 64.66, | H 7.88, | N 2.43 % |
| Gef.: | C 64.71, | H 7.97, | N 2.48 %. |

Beispiel 7

(A) In analoger Weise wie in Beispiel 1, Absatz (C) beschrieben, wurde aus 1-(3,4-Dimethoxyphenyl)-N-methyl-1-cyclopentanmethanamin und 1-Brom-2-methyl-3-chlorpropan das rac-N-(3-Chlor-2-methylpropyl)1-(3,4-dimethoxyphenyl)-N-methylcyclopentanmethanamin als ein dickflüssiges Oel (Sdp. 155°/390 Pa) erhalten, welches direkt in die nächste Stufe eingesetzt wurde.

(B) In analoger Weise wie in Beispiel 1, Absatz (D) beschrieben, wurde aus 2-(3,4-Dimethoxyphenyl)-m-dithian und rac-N-(3-Chlor-2-methylpropyl)-1-(3,4-dimethoxyphenyl)-N-methylcyclopentanmethanamin das rac-2-(3,4-Dimethoxyphenyl)-N-[[1-(3,4-dimethoxyphenyl)cyclopentyl]methyl]-β,N-dimethyl-m-dithian-2-propanamin-hydrochlorid als fester Schaum erhalten.

| $C_{31}H_{46}ClNO_4S_2$: | | | |
|---|---|---|---|
| Ber.: | C 61.42, | H 7.95, | N 2.05 % |
| Gef.: | C 61.09, | H 7.70, | N 1.87 %. |

Beispiel 8

In analoger Weise wie in Beispiel 1, Absatz (D) beschrieben, wurde aus 2-(3,4,5-Trimethoxyphenyl)-m-dithian und rac-N-(3-Chlor-2-methylpropyl)-1-(3,4-dimethoxyphenyl)-N-methylcyclopentanmethanamin das rac-N-[[1-(3,4-Dimethoxyphenyl)cyclopentyl]methyl]-β,N-dimethyl-2-(3,4,5-trimethoxyphenyl)-m-dithian-2-propanamin als dickflüssiges Oel erhalten.

| $C_{32}H_{47}NO_5S_2$: | | | |
|---|---|---|---|
| Ber.: | C 65.16, | H 8.03, | N 2.37 % |
| Gef.: | C 64.97, | H 8.12, | N 2.43 %. |

Beispiel 9

(A) 16,9 g (0,1 Mol) N-Methyl-2-(4-chlorphenyl)ethylamin wurden in 30 ml Dimethylformamid gelöst und mit 20,7 g (0,3 Mol) wasserfreiem Kaliumcarbonat versetzt. Das Gemisch wurde bei 5° gerührt und mit 12,6 ml (0,11 Mol) 1-Brom-2-methyl-3-chlorpropan versetzt, worauf weitere 4 Stunden bei 30° gerührt, das Lösungsmittel unter vermindertem Druck bei 30° eingedampft, und der Rückstand mit Wasser versetzt wurde. Das sich ausscheidende Oel wurde dreimal mit Ether extrahiert. Die organischen Extrakte wurden über Magnesiumsulfat getrocknet und unter reduziertem Druck eingedampft. Das zurückbleibende Oel wurde bei 135-140°/260 Pa destilliert, wobei man rac-4-Chlor-N-(3-chlor-2-methylpropyl)-N-methylphenethylamin als ein farbloses Oel erhielt, welches direkt in die nächste Stufe eingesetzt wurde. MS: 260 $(M)^+$.

(B) In einem Sulfierkolben wurden unter Argonbegasung 2,77 g (0,012 Mol) 2-(4-Chlorphenyl)-m-dithian und 10 ml abs. Tetrahydrofuran auf - 70° abgekühlt und mit 7,50 ml (0,012 Mol) Butyllithium in Hexan versetzt. Hierauf wurde das Gemisch 2 Stunden bei -20° gerührt. Eine Lösung von 2,50 g (0,0096 Mol) rac-4-Chlor-N-(3-chlor-2-methylpropyl)-N-methylphenethylamin in abs. Tetrahydrofuran wurde innerhalb 15 Minuten bei -70° dazugetropft, worauf das Gemisch 18 Stunden bei -20° und 3 Stunden bei Raumtemperatur stehen gelassen wurde. Die Reaktionslösung wurde dann auf Wasser gegossen und dreimal mit Ether extrahiert. Die etherischen Extrakte wurden über Magnesiumsulfat getrocknet und eingedampft. Das zurückbleibende Oel wurde an Kieselgel mit Toluol-Hexan (1:1) chromatographiert. Nach Eindampfen des Lösungsmittels und Erwärmen des Rückstandes bei 50° während 18 Stunden unter reduziertem Druck erhielt man rac-N-(4-Chlorphenethyl)-2-(4-chlorphenyl)-β,N-dimethyl-m-dithian-2-

propanaminals ein dickflüssiges Oel.

| $C_{23}H_{29}Cl_2NS_2$: | | | |
|---|---|---|---|
| Ber.: | C 60.78, | H 6.43, | N 3.08 % |
| Gef.: | C 60.81, | H 6.49, | N 3.04 %. |

Beispiel 10

In analoger Weise wie in Beispiel 9, Absatz (B) beschrieben, wurde aus 2-[(3,4-Methylendioxy)phenyl]-m-dithian und rac-4-Chlor-N-(3-chlor-2-methylpropyl)-N-methylphenethylamin das rac-N-(4-Chlorphenethyl)-$\beta$-methyl-2-[3,4-(methylendioxy)phenyl]-m-dithian-2-propanamin als ein dickflüssiges Oel erhalten.

| $C_{24}H_{30}ClNO_2S_2$: | | | |
|---|---|---|---|
| Ber.: | C 62.11, | H 6.52, | N 3.02 % |
| Gef.: | C 62.28, | H 6.38, | N 2.87 %. |

Beispiel 11

In analoger Weise wie in Beispiel 9, Absatz (B) beschrieben, wurde aus 2-(3,4-Dimethoxyphenyl)-m-dithian und rac-4-Chlor-N-(3-chlor-2-methylpropyl)-N-methylphenethylamin das rac-N-(4-Chlorphenethyl)-2-(3,4-dimethoxyphenyl)-$\beta$,N-dimethyl-m-dithian-2-propanamin als ein dickflüssiges Oel erhalten.

| $C_{25}H_{34}ClNO_2S_2$: | | | |
|---|---|---|---|
| Ber.: | C 62.54, | H 7.14, | N 2.92 % |
| Gef.: | C 62.55, | H 7.42, | N 2.86 %. |

Beispiel 12

(A) In analoger Weise wie in Beispiel 9, Absatz (A) beschrieben, wurde aus N-Methylhomoveratrylamin und rac-1-Brom-2-methyl-3-chlorpropan das rac-N-(3-Chlor-2-methylpropyl)-3,4-dimethoxy-N-methylphenethylamin als ein farbloses Oel erhalten, welches direkt in die nächste Stufe eingesetzt wurde. MS: 285 (M)+.

(B) In analoger Weise wie in Beispiel 9, Absatz (B) beschrieben, wurde aus 2-(4-Chlorphenyl)-m-dithian und rac-N-(3-Chlor-2-methylpropyl)-3,4-dimethoxy-N-methylphenethylamin das rac-2-(4-Chlorphenyl)-N-(3,4-dimethoxyphenethyl)-$\beta$,N-dimethyl-m-dithian-2-propanamin als dickflüssiges Oel erhalten.

| $C_{25}H_{34}ClNO_2S_2$: | | | |
|---|---|---|---|
| Ber.: | C 62.54, | H 7.14, | N 2.94 % |
| Gef.: | C 62.61, | H 7.15, | H 2.87 %. |

Beispiel 13

In analoger Weise wie in Beispiel 9, Absatz (B) beschrieben, wurde aus 2-(4-Tolyl)-m-dithian und rac-N-(3-Chlor-2-methylpropyl)-3,4-dimethoxy-N-methylphenethylamin das rac-N-(3,4-Dimethoxyphenethyl)-$\beta$,N-dimethyl-2-(4-tolyl)-m-dithian-2-propanamin als ein dickflüssiges Oel erhalten.

| $C_{26}H_{37}NO_2S_2$: | | | |
| --- | --- | --- | --- |
| Ber.: | C 67.93, | H 8.11, | N 3.05 % |
| Gef.: | C 68.06, | H 8.23, | N 3.01 %. |

Beispiel 14

In analoger Weise wie in Beispiel 9, Absatz (B) beschrieben, wurde aus 2-(2-Naphthyl)-m-dithian und rac-N-(3-Chlor-2-methylpropyl)-3,4-dimethoxy-N-methylphenethylamin ein farbloses Oel erhalten, welches mit überschüssigem Chlorwasserstoff in abs. Essigester in das als weisser Schaum anfallende rac-N-(3,4-Dimethoxyphenethyl)-β,N-dimethyl-2-(2-naphthyl)-m-dithian-2-propanamin-hydrochlorid überführt wurde.

| $C_{29}H_{37}NO_2S_2$ (freie Base): | | | |
| --- | --- | --- | --- |
| Ber.: | C 70.26, | H 7.52, | N 2.83 % |
| Gef.: | C 70.63, | H 7.74, | N 2.64 %. |

Beispiel 15

(A) Eine Lösung von 10,93 g (0,056 Mol) N-Methylhomoveratrylamin in 50 ml abs. Dimethylformamid wurde bei 5 ° mit 15,7 g Kaliumcarbonat und 13,6 g (0,056 Mol) (R)-(-)-3-Benzyloxy-2-methylpropylbromid [Helv. Chim. Acta 60, 940, (1977)] versetzt. Das Gemisch wurde 18 Stunden bei Raumtemperatur gerührt und dann unter vermindertem Druck eingedampft. Der Rückstand wurde in einem Gemisch von Wasser und Essigester aufgenommen. Die organischen Extrakte wurden über Magnesiumsulfat getrocknet und unter reduziertem Druck eingedampft. Der Rückstand wurde bei 218°/260 Pa destilliert. Das als farbloses Oel erhaltene N-[(S)-3-(Benzyloxy)-2-methylpropyl]-3,4-dimethoxy-N-methylphenethylamin wurde direkt in die nächste Stufe eingesetzt. $[\alpha]^{20}_D$ = +7.3° (c = 1, Ethanol).

(B) Eine Lösung von 12,8 g (0,0358 Mol) N-[(S)-3-(Benzyloxy)-2-methylpropyl]-3,4-dimethoxy-N-methylphenethylamin in 50 ml abs. Methanol und 35,8 ml 2 N Salzsäure wurde mit 1,28 g 5 %igem Palladium/Kohle versetzt und unter Atmosphärendruck bei Raumtemperatur hydriert. Die abfiltrierte Lösung wurde unter vermindertem Druck eingedampft. Der Rückstand wurde in Essigester aufgenommen und mit einer 5 %igen Natriumcarbonat-Lösung gewaschen. Nach dem Trocknen und Eindampfen des organischen Lösungsmittels wurde der ölige Rückstand bei 155°/130 Pa destilliert. Man erhielt (S)-3-[(3,4-Dimethoxyphenethyl)methylamino]-2-methyl-1-propanol als ein farbloses Oel, $[\alpha]^{20}_D$ = +12,6° (c = 1, Ethanol).

| $C_{15}H_{25}NO_3$: | | | |
| --- | --- | --- | --- |
| Ber.: | C 67.38, | H 9.43, | N 5.24 % |
| Gef.: | C 66.92, | H 9.52, | N 5.33 %. |

(C) Eine Lösung von 5,83 g (0,0218 Mol) (S)-3-[(3,4-Dimethoxyphenethyl)methylamino]-2-methyl-1-propanol in 50 ml Methylenchlorid wurde bei Raumtemperatur mit 2,4 ml Thionylchlorid versetzt, über Nacht stehen gelassen und dann unter vermindertem Druck eingedampft. Der Rückstand wurde zwischen Ether und einer Natriumcarbonat-Lösung (5 %) verteilt. Die organischen Extrakte wurden über Magnesiumsulfat getrocknet und unter vermindertem Druck eingedampft. Aus dem als Oel anfallenden N-[(S)-3-Chlor-2-methylpropyl]-3,4-dimethoxy-N-methylphenethylamin wurde mittels Chlorwasserstoff in Essigester das N-[(S)-3-Chlor-2-methylpropyl]-3,4-dimethoxy-N-methylphenethylamin-hydrochlorid als eine feste Substanz mit einem Schmelzpunkt von 109-110° erhalten, $[\alpha]^{20}_D$ = -3,5° (c = 1, Ethanol)

| $C_{15}H_{24}ClNO_2 \cdot HCl$: | | | |
|---|---|---|---|
| Ber.: | C 55.90, | H 7.82, | N 4.34 % |
| Gef.: | C 55.87, | H 7.95, | N 4.31 %. |

(D) In analoger Weise wie in Beispiel 1, Absatz (D) beschrieben, wurde aus 2-(3,4-Dimethoxyphenyl)-m-dithian und N-[(S)-3-Chlor-2-methylpropyl]-3,4-dimethoxy-N-methylphenethylamin ein farbloses Oel erhalten, welches mit überschüssigem Chlorwasserstoff in abs. Essigester in das (R)-N-(3,4-Dimethoxyphenethyl)-2-(3,4-dimethoxyphenyl)-β,N-dimethyl-m-dithian-2-propanamin-hydrochlorid in Form eines weissen Schaums übergeführt wurde.

| $C_{27}H_{39}NO_4S_2 \cdot HCl$: | | | |
|---|---|---|---|
| Ber.: | C 59.81, | H 7.44, | N 2.58 % |
| Gef.: | C 59.47, | H 7.54, | N 2.77 %. |

Das entsprechende Oxalat (1:1) vom Schmelzpunkt 131-132° wurde aus Acetonitril kristallisiert, $[\alpha]^{25}_D$ = -19.6° (c = 1, Ethanol).

| $C_{27}H_{39}NO_4S_2 \cdot C_2H_2O_4$: | | | |
|---|---|---|---|
| Ber.: | C 58.47, | H 6.94, | N 2.35 % |
| Gef.: | C 58.33, | H 6.94, | N 2.31 %. |

Das entsprechende Amidosulfat (1:1) wurde nach üblicher Lyophilisierung einer wässerigen Lösung einer äquivalenten Menge der Base und Sulfaminsäure erhalten.

| $C_{27}H_{39}NO_4S_2 \cdot H_3NO_3S$: | | | |
|---|---|---|---|
| Ber.: | C 53.80, | H 7.02, | N 4.65 % |
| Gef.: | C 53.76, | H 7.35, | N 4.29 %. |

Eine Lösung von 679 mg (R)-N-(3,4-Dimethoxyphenethyl)-2-(3,4-dimethoxyphenyl)-β,N-dimethyl-m-dithian-2-propanamin und 508 mg Ascorbinsäure in 5 ml Wasser wurde nach üblicher Weise lyophilisiert. Das erhaltene (1:2)-Ascorbat wurde 2 Tage bei 50° im Hochvakuum getrocknet, Smp. 75°, $[\alpha]^{25}_D$ = +24.7° (c = 1, Ethanol).

| $C_{27}H_{39}NO_4S_2 \cdot 2C_6H_8O_6$: | | | | |
|---|---|---|---|---|
| Ber.: | C 54.60, | H 6.46, | N 1.63, | S 7.47 % |
| Gef.: | C 54.22, | H 6.37, | N 1.80, | S 7.24%. |

Beispiel 16

(A) In analoger Weise wie in Beispiel 15, Absatz (A) beschrieben, wurde aus N-Methylhomoveratrylamin und (S)-(+)-3-Benzyloxy-2-methylpropylbromid[Helv. Chim. Acta 60, 940, (1977)] das N-[(R)-3-(Benzyloxy)-2-methylpropyl]-3,4-dimethoxy-N-methylphenethylamin als ein farbloses Oel erhalten, welches direkt in die nächste Stufe eingesetzt wurde. $[\alpha]^{20}_D$ = -7.0° (c = 0.9, Ethanol).

(B) In analoger Weise wie in Beispiel 15, Absatz (B) beschrieben, wurde aus N-[(R)-3-(Benzyloxy)-2-methylpropyl]-3,4-dimethoxy-N-methylphenethylamin das (R)-3-[(3,4-Dimethoxyphenethyl)methylamino]-2-methyl-1-propanolals ein farbloses Oel erhalten, $[\alpha]^{20}_D$ = -12.2° (c = 1, Ethanol).

| $C_{15}H_{25}NO_3$: | | | |
|---|---|---|---|
| Ber.:<br>Gef.: | C 67.38,<br>C 66.98, | H 9.43,<br>H 9.63, | N 5.24 %<br>N 5.38 %. |

(C) In analoger Weise wie in Beispiel 15, Absatz (C) beschrieben, wurde aus (R)-3-[(3,4-Dimethoxyphenethyl)methylamino]-2-methyl-1-propanol das N-[(R)-3-Chlor-2-methylpropyl]-3,4-dimethoxy-N-methylphenethylamin als ein Oel erhalten, welches in das entsprechende Hydrochlorid, eine feste Substanz vom Schmelzpunkt 109-110° (aus Essigester) überführt wurde, $[\alpha]^{20}_D$ = +3,5° (c= 1, Ethanol).

| $C_{15}H_{24}ClNO_2 \cdot HCl$: | | | |
|---|---|---|---|
| Ber.:<br>Gef.: | C 55.90,<br>C 55.83, | H 7.82,<br>H 7.91, | N 4.35 %<br>N 4.31 %. |

(D) In analoger Weise wie in Beispiel 1, Absatz (D) beschrieben, wurde aus 2-(3,4-Dimethoxyphenyl)-m-dithian und N-[(R)-3-Chlor-2-methylpropyl]-3,4-dimethoxy-N-methylphenethylamin das (S)-N-(3,4-Dimethoxyphenethyl)-2-(3,4-dimethoxyphenyl)-β,N-dimethyl-m-dithian-2-propanamin-hydrochlorid als weisser Schaum erhalten.

| $C_{27}H_{39}NO_4S_2 \cdot HCl$: | | | |
|---|---|---|---|
| Ber.:<br>Gef.: | C 59.81,<br>C 59.69, | H 7.44,<br>H 7.59, | N 2.58 %<br>N 2.58 %. |

Das entsprechende Oxalat (1:1) vom Schmelzpunkt 131-132° wurde aus Acetonitril kristallisiert, $[\alpha]^{25}_D$ = +18.9° (c = 1, Ethanol).

| $C_{27}H_{39}NO_4S_2 \cdot C_2H_2O_4$: | | | | |
|---|---|---|---|---|
| Ber.:<br>Gef.: | C 58.47,<br>C 58.41, | H 6.94,<br>H 6.76, | N 2.35,<br>N 2.15, | S 10.76 %<br>S 10.79 %. |

Das entsprechende Amidosulfat (1:1) wurde nach üblicher Lyophilisierung einer wässerigen Lösung der Base und Sulfaminsäure erhalten.

| $C_{27}H_{39}NO_4S_2 \cdot H_3NO_3S$: | | | |
|---|---|---|---|
| Ber.:<br>Gef.: | C 53.80,<br>C 53.76, | H 7.02,<br>H 7.35, | N 4.65%<br>N 4.29%. |

Eine Lösung aus 15.55 g (30,7 mMol) (S)-N-(3,4-Dimethoxyphenethyl)-2-(3,4-dimethoxyphenyl)-β,N-dimethyl-m-dithian-2-propanamin und 10.83 g (61.5 mMol) Ascorbinsäure in 75 ml Wasser wurde über Norit filtriert und nach üblicher Weise lyophilisiert. Das erhaltene (1:2)-Ascorbat wurde 2 Tage bei 50° im Hochvakuum getrocknet, Smp. 75°, $[\alpha]^{25}_D$ = +46.1° (c = 1, Ethanol).

| $C_{27}H_{39}NO_4S_2 \cdot 2C_6H_8O_6$: | | | | |
|---|---|---|---|---|
| Ber.:<br>Gef.: | C 54.60,<br>C 54.43, | H 6.46,<br>H 6.58, | N 1.63,<br>N 1.58, | S 7.47%<br>S 7.14%. |

Beispiel 17

(A) In analoger Weise wie in Beispiel 15, Absatz (A) beschrieben, wurde aus 1-(3,4-Dimethoxyphenyl)-

cyclopentanmethanamin und (S)-(+)-3-Benzyloxy-2-methylpropylbromid das (R)-(-)-N-(3-Benzyloxy-2-methylpropyl)-1-(3,4-dimethoxyphenyl)-N-methylcyclopentanmethanamin als ein farbloses Oel erhalten, welches direkt in die nächste Stufe eingesetzt wurde. $[\alpha]^{20}_D$ = - 2,0° (c = 1, Ethanol).

(B) In analoger Weise wie in Beispiel 15, Absatz (B) beschrieben, wurde aus (R)-(-)-N-(3-Benzyloxy-2-methylpropyl)-1-(3,4-dimethoxyphenyl)-N-methylcyclopentanmethanamin das (R)-3-[[[1-(3,4-Dimethoxyphenyl)cyclopentyl]methyl]methylamino]-2-methyl-1-propanol als ein farbloses Oel erhalten, welches direkt in die nächste Stufe eingesetzt wurde. $[\alpha]^{20}_D$ = -27,7° (c = 1, Ethanol).

(C) In analoger Weise wie in Beispiel 15, Absatz (C) beschrieben, wurde aus (R)-3-[[[1-(3,4-Dimethoxyphenyl)cyclopentyl]methyl]methylamino]-2-methyl-1-propanol das (R)-(-)-N-(3-Chlor-2-methylpropyl)-1-(3,4-dimethoxyphenyl)-N-methylcyclopentanmethanamin als ein farbloses Oel erhalten, welches direkt in die nächste Stufe eingesetzt wurde. $[\alpha]^{20}_D$ = -21,5° (c = 1, Ethanol).

(D) In analoger Weise wie in Beispiel 1, Absatz (D) beschrieben, wurde aus 2-(3,4-Dimethoxyphenyl)-m-dithian und (R)-(-)-N-(3-Chlor-2-methylpropyl)-1-(3,4-dimethoxyphenyl)-N-methylcyclopentanmethanamin das (S)-(+)-2-[3,4-Dimethoxyphenyl-N-[[(1-(3,4-dimethoxyphenyl)cyclopentyl]methyl]-β,N-dimethyl-m-dithian-2-propanamin als ein dickflüssiges Oel erhalten.

| $C_{31}H_{45}NO_4S_2$: | | | |
|---|---|---|---|
| Ber.: | C 66.51, | H 8.10, | N 2.50 % |
| Gef.: | C 66.91, | H 8.30, | N 2.36 %. |

Beispiel 18

(A) Zu einer Lösung von 198 g (1.1 Mol) Homoveratronitril in 500 ml Diäthylcarbonat wurden nach und nach 25.7 g (1.1 Mol aeq.) Natrium in Stückchen so zugegeben, dass die Temperatur bei ungefähr 100° gehalten wurde. Danach wurde das Reaktionsgemisch 1 Stunde zum Rückfluss erhitzt, unter vermindertem Druck eingedampft, mit gekühltem Wasser versetzt, mit 70 ml Eisessig angesäuert und mit Aether extrahiert. Die vereinigten organischen Extrakte wurden getrocknet und eingedampft. Das zurückbleibende Oel wurde im Hochvakuum destilliert, wobei 3,4-Dimethoxyphenylcyanessigsäureäthylester erhalten wurde, Sdp. 170-172°/70 Pa. HPLC-Reinheit >99%.

(B) Zu einer Suspension von 84,5 g (0.753 Mol) Kalium-tert-butylat in 300 ml abs. Dimethylformamid wurden 124.5 g (0.5 Mol) 3,4-Dimethoxyphenylcyanessigsäureäthylester in 160 ml abs. Dimethylformamid bei 15-20° unter Rühren gegeben. Nach einer Stunde wurden 83.5 ml 1-Brom-3-chlorpropan langsam zugetropft, wobei die Temperatur 15-20° betrug. Nach 16 Stunden wurde das Reaktionsgemisch im Hochvakuum eingedampft. Der Rückstand wurde zwischen Aether und einer wässerigen gesättigten Lösung von Ammoniumchlorid verteilt. Die vereinigten organischen Extrakte wurden getrocknet und unter vermindertem Druck eingedampft. Der ölige Rückstand wurde im Hochvakuum destilliert, wobei 145.7 g [γ-Chlorpropyl-3,4-dimethoxyphenyl]cyanessigsäureäthylester erhalten wurden, Sdp. 195-201°/80 Pa.

(C) Eine Lösung von 145.7 g (0.44 Mol) [γ-Chlorpropyl-3,4-dimethoxyphenyl]cyanessigsäureäthylester in 100 ml abs. Tetrahydrofuran wurde innerhalb von 30 Minuten unter leichten Kühlung und heftigen Rühren, zu einer Suspension von 50.2 g (0.45 Mol) Kalium-tert-butylat in 150 ml abs. Tetrahydrofuran gegeben, wobei sich die Reaktionslösung violett verfärbte. Nach einer Stunde wurde die Lösung unter vermindertem Druck eingedampft, und der Rückstand zwischen Aether und einer gesättigten Ammoniumchloridlösung verteilt. Die organischen Extrakte wurden getrocknet und unter vermindertem Druck eingedampft. Der ölige Rückstand wurde im Hochvakuum destilliert, wobei 1-(3,4-Dimethoxyphenyl)-cyclobutancarbonitril erhalten wurde, Sdp. 162-165°/80 Pa.

(D) Zu einer Lösung von 84.0 g (0.387 Mol) 1-(3,4-Dimethoxyphenyl)cyclobutancarbonitril in 100 ml Aethylenglykol wurden 21.7 g (0.387 Mol) Kaliumhydroxid gegeben, und das Gemisch 18 Stunden auf 150° erhitzt. Die Reaktionslösung wurde dann unter Hochvakuum auf 40 ml eingeengt, mit kaltem Wasser verdünnt und mit Aether extrahiert. Die wässerigen Lösungen wurden vereinigt, mit 3N Salzsäure angesäuert und viermal mit Aether extrahiert. Die Aetherextrakte wurden getrocknet und unter vermindertem Druck eingedampft. So erhielt man 1-(3,4-Dimethoxyphenyl)cyclobutancarbonsäureals ein farbloses Oel mit einer nach HPLC bestimmten Reinheit von 95.5%.

Das entsprechende Dicyclohexylaminsalz (1:1) konnte als ein weisser Festkörper vom Schmelzpunkt 119-120° aus Aether erhalten werden.

| $C_{25}H_{39}NO_4$: | | | |
|---|---|---|---|
| Ber.: | C 71.91, | H 9.41, | N 3.35 % |
| Gef.: | C 71.70, | H 9.55, | N 3.32 %. |

(E) Eine Lösung der obigen Säure (66.1 g, 0.28 Mol) in 50 ml Toluol wurde mit 41 ml Thionylchlorid versetzt und 2 Stunden zum Rückfluss erhitzt und danach unter vermindertem Druck eingedampft. Der Rückstand wurde mit 50 ml Toluol verdünnt, und die Lösung nochmals unter vermindertem Druck eingedampft. Das zurückbleibende Oel wurde in 200 ml abs. Aether gelöst und langsam unter Kühlung bei ungefähr 5° mit 31 ml bei -20° kondensiertem Methylamin versetzt. Das Gemisch wurde über Nacht bei Raumtemperatur gerührt und dann zwischen Aether und 1N Natronlauge verteilt. Die vereinigten organischen Extrakte wurden getrocknet und unter vermindertem Druck eingedampft. So erhielt man 1-(3,4-Dimethoxyphenyl)-N-methylcyclobutancarbonsäureamid mit einer nach HPLC bestimmten Reinheit von 91.6%, welches direkt ohne weitere Reinigung in die nächste Stufe eingesetzt wurde.

(F) Zu einer Suspension von 15.5 g Lithiumaluminiumhydrid in 400 ml abs. Tetrahydrofuran wurde unter Rühren eine Lösung von 64.4 g (0.26 Mol) des obigen N-Methylamids in 200 ml abs. Tetrahydrofuran getropft. Nach 24-stündigem Erhitzen zum Rückfluss wurden nochmals 10.0 g Lithiumaluminiumhydrid zugegeben, und die Suspension nochmals 2,5 Stunden zum Rückfluss erhitzt. Nach dem Abkühlen auf 5° wurde die Suspension vorsichtig mit einer gesättigten, wässrigen Natriumsulfatlösung versetzt. Der dabei entstandene Niederschlag wurde abfiltriert und mehrere Male mit Tetrahydrofuran gewaschen. Die organischen Lösungen wurden eingedampft, und der ölige Rückstand im Hochvakuum destilliert. So erhielt man 1-(3,4-Dimethoxyphenyl)-N-methylcyclobutanmethanamin als ein farbloses Oel, Sdp. 123-124°/8 Pa.

Das entsprechende Hydrochlorid wurde aus Chlorwasserstoff/Dioxan mit einem Schmelzpunkt von 233-234° erhalten.

(G) In analoger Weise wie in Beispiel 1, Absatz (C) beschrieben, wurde aus 1-(3,4-Dimethoxyphenyl)-N-methylcyclobutanmethanamin und 1-Brom-3-chlorpropan das N-(3-Chlorpropyl)-1-(3,4-dimethoxyphenyl)-cyclobutanmethanamin als dickflüssiges Oel (Sdp. 170°/260) Pa erhalten, welches direkt in die nächste Stufe eingesetzt wurde.

(H) In analoger Weise wie in Beispiel 1, Absatz (D) beschrieben, wurde aus 2-(3,4-Dimethoxyphenyl)-m-dithian und N-(3-Chlorpropyl)-1-(3,4-dimethoxyphenyl)cyclobutanmethanamin das 2-(3,4-Dimethoxyphenyl)-N-[[1-(3,4-dimethoxyphenyl)cyclobutyl]methyl]-N-methyl-m-dithian-2-propanamin als dickflüssiges Oel erhalten.

| $C_{29}H_{41}NO_4S_2$: | | | |
|---|---|---|---|
| Ber.: | C 65.50, | H 7.77, | N 2.63 % |
| Gef.: | C 65.28, | H 7.90, | N 2.53 %. |

Beispiel 19

In analoger Weise wie in Beispiel 1, Absatz (D) beschrieben, wurde aus 2-(3,4,5-Trimethoxyphenyl)-m-dithian und N-(3-Chlorpropyl)-1-(3,4-dimethoxyphenyl)cyclobutanmethanamin das N-[[1-(3,4-Dimethoxyphenyl)cyclobutyl]methyl]-N-methyl-2-(3,4,5-trimethoxyphenyl)-m-dithian-2-propanaminhydrochlorid als Festkörper erhalten.

| $C_{30}H_{43}NO_5S_2 \cdot HCl$: | | | |
|---|---|---|---|
| Ber.: | C 60.23, | H 7.41, | N 2.34 % |
| Gef.: | C 60.19, | H 7.44, | N 2.35 %. |

Beispiel 20

In analoger Weise wie in Beispiel 1, Absatz (D) beschrieben, wurde aus 2-(3,4-Ethylendioxyphenyl)-m-dithian und N-(3-Chlorpropyl)-1-(3,4-dimethoxyphenyl)cyclobutanmethanamin das 2-(1,4-Benzodioxan-6-yl)-N-[[1-(3,4-dimethoxyphenyl)cyclobutyl]methyl]-N-methyl-m-dithian-2-propanaminhydrochlorid als Festkörper erhalten.

| $C_{29}H_{39}NO_4S_2 \cdot HCl$: | | | |
|---|---|---|---|
| Ber.: | C 61.52, | H 7.12, | N 2.47 % |
| Gef.: | C 60.95, | H 7.60, | N 2.41 %. |

Beispiel 21

In analoger Weise wie in Beispiel 1, Absatz (D) beschrieben, wurde aus 2-(3,4-Dimethylphenyl)-m-dithian und N-(3-Chlorpropyl)-1-(3,4-dimethoxyphenyl)cyclobutanmethanamin das N-[[1-(3,4-Dimethoxyphenyl)cyclobutyl]methyl]-2-(3,4-dimethylphenyl)-N-methyl-m-dithian-2-propanaminhydrochlorid als Festkörper erhalten.

| $C_{29}H_{41}NO_2S_2 \cdot HCl$: | | | |
|---|---|---|---|
| Ber.: | C 64.96, | H 7.90, | N 2.61 % |
| Gef.: | C 64.31, | H 7.97, | N 2.45 %. |

Beispiel 22

In analoger Weise wie in Beispiel 1, Absatz (D) beschrieben, wurde aus 2-(3,5-Dimethoxyphenyl)-m-dithian und N-(3-Chlorpropyl)-1-(3,4-dimethoxyphenyl)cyclobutanmethanamin das N-[[1-(3,4-Dimethoxyphenyl)cyclobutyl]methyl]-2-(3,5-dimethoxyphenyl)-N-methyl-m-dithian-2-propanamin als dickflüssiges Oel erhalten.

| $C_{29}H_{41}NO_4S_2$: | | | |
|---|---|---|---|
| Ber.: | C 65.50, | H 7.77, | N 2.63 % |
| Gef.: | C 64.98, | H 7.82, | N 2.56 %. |

Beispiel 23

In analoger Weise wie in Beispiel 1, Absatz (D) beschrieben, wurde aus 2-(4-Chlorphenyl)-m-dithian und N-(3-Chlorpropyl)-1-(3,4-dimethoxyphenyl)cyclobutanmethanamin das 2-(4-Chlorphenyl)-N-[[1-(3,4-dimethoxyphenyl)cyclobutyl]methyl]-N-methyl-m-dithian-2-propanamin-hydrochlorid als Festkörper erhalten.

| $C_{27}H_{36}ClNO_2S_2 \cdot HCl$: | | | |
|---|---|---|---|
| Ber.: | C 59.76, | H 6.87, | N 2.58 % |
| Gef.: | C 59.75, | H 7.05, | N 2.55 %. |

Beispiel 24

(A) Ein Gemisch von 40.6 g (0.173 Mol) 1-(3,4-Dimethoxyphenyl)-N-methylcyclobutanmethanamin und 29.8 g (0.123 Mol) (S)-(+)-3-Benzyloxy-2-methylpropylbromid [Helv. Chim. Acta 60, 940 (1977)] wurde 2,5 Stunden unter gutem Rühren auf 120° erhitzt. Nach dem Abkühlen des Gemisches auf Raumtemperatur wurden 200 ml Aether zugegeben. Der dabei gebildete Niederschlag wurde abgenutscht und mit Aether gewaschen. Die ätherische Lösung wurde dreimal mit 1N Salzsäure extrahiert, die sauren Extrakte mit 1N Natronlauge basisch gestellt und dreimal mit Aether extrahiert. Die Aetherextrakte wurden getrocknet, eingedampft und an Kieselgel mit n-Hexan/Aether (1:1) als Eluierungsmittel chromatographiert. So wurde N-[(R)-3-(Benzyloxy)-2-methylpropyl]-3,4-dimethoxyphenyl-N-methylcyclobutanmethanamin als ein farbloses Oel erhalten, $[\alpha]^{25}_{D}$ = -9,2° (c = 0.6, Ethanol).

| $C_{25}H_{35}NO_3$: | | | |
|---|---|---|---|
| Ber.: | C 75.53, | H 8.87, | N 3.52 % |
| Gef.: | C 75.26, | H 8.96, | N 3.49 %. |

(B) Eine Lösung von 35.7 g (0.09 Mol) N-[(R)-3-(Benzyloxy)-2-methylpropyl]-3,4-dimethoxyphenyl-N-methylcyclobutanmethanamin und 10.4 ml Benzylchlorid in 300 ml abs. Ethanol wurde unter Argon mit 3.6 g 10%igem Palladium/Kohle versetzt und unter Atmosphärendruck bei Raumtemperatur bis zur beendeten Wasserstoffaufnahme hydriert. Nach dem Abnutschen des Katalysators wurde die farblose Lösung unter vermindertem Druck eingedampft. Der ölige Rückstand wurde 24 Stunden im Hochvakuum von organischen Lösungsmitteln befreit. So erhielt man das (R)-3-[[[1-(3,4-Dimethoxyphenyl)cyclobutyl]-methyl]methylamino]-2-methyl-1-propanol als ein farbloses Oel, $[\alpha]^{25}_{D}$ = -32,2° (c= 1, Ethanol).

| $C_{18}H_{29}NO_3$: | | | |
|---|---|---|---|
| Ber.: | C 70.32, | H 9.51, | N 4.56 % |
| Gef.: | C 69.91, | H 9.54, | N 4.55 %. |

(C) Eine Lösung von 26.7g (0.087 Mol) (R)-3-[[[1-(3,4-Dimethoxyphenyl)cyclobutyl]methyl]methylamino]-2-methyl-1-propanol in 160 ml abs. Methylenchlorid wurde zuerst mit 87 ml 1N Salzsäure in abs. Methylenchlorid und dann mit 12,6 ml Thionylchlorid versetzt. Diese Lösung wurde über Nacht bei Raumtemperatur gerührt und danach unter vermindertem Druck eingedampft. Der Rückstand wurde zwischen Aether und einer 5%igen Natriumcarbonatlösung verteilt, und die organischen Extrakte getrocknet und unter vermindertem Druck eingedampft. Der ölige Rückstand wurde im Hochvakuum bei Raumtemperatur von organischen Lösungsmitteln befreit. So wurde N-[(R)-3-Chlor-2-methylpropyl]-1-(3,4-dimethoxyphenyl)-N-methylcyclobutanmethanamin als farbloses Oel und mit einer nach HPLC bestimmten Reinheit von 98.8% erhalten, $[\alpha]^{25}_{D}$ = -23,2° (c = 1, Ethanol).

| $C_{18}H_{28}ClNO_2$: | | | | |
|---|---|---|---|---|
| Ber.: | C 66.34, | H 8.66, | N 4.30, | Cl 10.88 % |
| Gef.: | C 66.44, | H 8.90, | N 4.30, | Cl 10.56 %. |

(D) In einem Sulfierkolben wurde unter Begasung mit getrocknetem Argon eine Lösung von 34,6 g (0.135 Mol) 2-(3,4-Dimethoxyphenyl)-m-dithian in 200 ml abs. Tetrahydrofuran auf -78° abgekühlt und innerhalb von 15 Minuten tropfenweise mit 84 ml (0.135 Mol) einer Lösung von Butyllithium in Hexan versetzt, wobei die Temperatur bei -78° gehalten wurde. Danach wurde die Lösung bei -20° 1 Stunde gerührt und dann wieder auf -78° abgekühlt und mit einer Lösung von 29,32 g (0.09 Mol) N-[(R)-3-Chlor-2-methylpropyl]-1-(3,4-dimethoxyphenyl)-N-methylcyclobutanmethanamin in 75 ml abs. Tetrahydrofuran tropfenweise versetzt. Nach 16 Stunden bei -20° und 2 Stunden bei Raumtemperatur wurde die Lösung wieder auf -10° abgekühlt und mit 100 ml einer gesättigten Ammoniumchloridlösung versetzt. Das Gemisch wurde unter vermindertem Druck auf ein Volumen von 120 ml eingeengt und danach mit Aether extrahiert. Die ätherischen Extrakte wurden viermal mit 3N Salzsäure und zweimal mit Wasser extrahiert. Die wässerigen Extrakte wurden erst mit Eis und dann mit 6N Natronlauge bis zum pH 13 versetzt und mit Aether viermal extrahiert. Die ätherischen Extrakte wurden über Magnesiumsulfat getrocknet, filtriert und unter vermindertem Druck eingedampft. Das zurückbleibende Oel wurde an Kieselgel 60 mit Chloroform/Ethanol/Essigsäure (190:10:5) als Eluierungsmittel chromatographiert. Nach Eindampfen des Lösungsmittelgemisches wurde ein farbloses Oel erhalten, welches im Hochvakuum bei Raumtemperatur 24 Stunden von organischen Lösungsmitteln befreit wurde. Das so erhaltene (S)-(+)-2-(3,4-Dimethoxyphenyl)-N-[[1-(3,4-dimethoxyphenyl)cyclobutyl]methyl]-β,N-dimethyl-m-dithian-2-propanamin besitzt einen $[\alpha]^{25}_{D}$ -Wert von +3,75° (c = 0.4, Ethanol).

| $C_{30}H_{43}NO_4S_2$: | | | |
|---|---|---|---|
| Ber.: | C 66.02, | H 7.94, | N 2.57 % |
| Gef.: | C 66.00, | H 7.95, | N 2.47 %. |

Die Base wurde in das entsprechende Hydrochlorid, einen farblosen Schaum, übergeführt, $[\alpha]^{25}_D$ = +4.4° (c = 1, Ethanol).

| $C_{30}H_{43}NO_4S_2 \cdot HCl$: | | | | |
|---|---|---|---|---|
| Ber.: | C 61.08, | H 7.62, | N 2.41, | S 11.01 % |
| Gef.: | C 61.31, | H 7.57, | N 2.38, | S 10.91 %. |

546 mg (1 mMol) der obengenannten Base und 98 mg (1 mMol) Sulfaminsäure wurden in 10 ml Wasser bei Raumtemperatur gerührt bis eine klare Lösung entstand. Diese Lösung wurde bei -20° gefroren und im Hochvakuum lyophilisiert. Der feste Rückstand wurde 24 Stunden im Hochvakuum getrocknet, wobei das (S)-2-(3,4-Dimethoxyphenyl)-N-[[1-(3,4-dimethoxyphenyl)cyclobutyl]methyl]-β,N-dimethyl-m-dithian-2-propanamid-amidosulfat (1:1) erhalten wurde, $[\alpha]^{25}_D$ = +2.2° (c = 1, Ethanol).

| $C_{30}H_{43}NO_4S_2 \cdot H_3NO_3S$: | | | | |
|---|---|---|---|---|
| Ber.: | C 56.05, | H 7.21, | N 4.36, | S 14.96 % |
| Gef.: | C 55.35, | H 7.32, | N 4.64, | S 15.14 %. |

Beispiel 25

(A) In analoger Weise wie in Beispiel 24, Absatz (A) beschrieben, wurde aus 1-(3,4-Dimethoxyphenyl)-N-methylcyclobutanmethanamin und (R)-(-)-3-Benzyloxy-2-methylpropylbromid [Helv. Chim. Acta 60, 940-(1977)] das N-[(S)-3-(Benzyloxy)-2-methylpropyl]-3,4-dimethoxyphenyl-N-methylcyclobutanmethanamin als ein farbloses Oel mit einer nach HPLC bestimmten Reinheit von 97.5% erhalten, $[\alpha]^{25}_D$ = +9.4° (c = 0.6, Ethanol).
(B) In analoger Weise wie in Beispiel 24, Absatz (B) beschrieben, wurde aus N-[(S)-3-(Benzyloxy)-2-methylpropyl]-3,4-dimethoxyphenyl-N-methylcyclobutanmethanamin, Wasserstoff und Palladium auf Kohle das (S)-3-[[[1-(3,4-Dimethoxyphenyl)cyclobutyl]methyl]methylamino]-2-methyl-1-propanol als ein farbloses Oel erhalten, $[\alpha]^{25}_D$ = +29.9° (c = 0.7, Ethanol).
(C) In analoger Weise wie in Beispiel 24, Absatz (C) beschrieben, wurde aus (S)-3[[[1-(3,4-Dimethoxyphenyl)cyclobutyl]methyl]methylamino]-2-methyl-1-propanol und Thionylchlorid das N-[(S)-3-Chlor-2-methylpropyl]-1-(3,4-dimethoxyphenyl)-N-methylcyclobutanmethanamin als ein farbloses Oel mit einer nach HPLC bestimmten Reinheit von ~100% erhalten, $[\alpha]^{25}_D$ = +21.9° (c = 0.9, Ethanol), welches direkt in die nächste Stufe eingesetzt wurde.
(D) In analoger Weise wie in Beispiel 24, Absatz (D) beschrieben, wurde aus 2-(3,4-Dimethoxyphenyl)-m-dithian, Butyllithium und N-[(S)-3-Chlor-2-methylpropyl]-1-(3,4-dimethoxyphenyl)-N-methylcyclobutanmethanamin das (R)-(-)-2-(3,4-Dimethoxyphenyl)-N-[[1-(3,4-dimethoxyphenyl)cyclobutyl]methyl]-β,N-dimethyl-m-dithian-2-propanamid erhalten, $[\alpha]^{25}_D$ = -3.3° (c = 3, Ethanol).

| $C_{30}H_{43}NO_4S_2$: | | | | |
|---|---|---|---|---|
| Ber.: | C 66.02, | H 7.94, | N 2.57, | S 11.75 % |
| Gef.: | C 66.17, | H 8.05, | N 2.48, | S 11.68 %. |

Die Base wurde in das entsprechende Hydrochlorid, einen farblosen Schaum, übergeführt.

| $C_{30}H_{43}NO_4S_2 \cdot HCl$: | | | | | |
|---|---|---|---|---|---|
| Ber.: | C 61.88, | H 7.62, | N 2.41, | S 11.01, | Cl 6.09 % |
| Gef.: | C 61.40, | H 7.94, | N 2.32, | S 10.73, | Cl 6.66 %. |

In analoger Weise wie in Beispiel 24, Absatz (D), letzter Absatz beschrieben, wurde aus der oben beschriebenen Base und Sulfaminsäure das (R)-2-(3,4-Dimethoxyphenyl)-N-[[1-(3,4-dimethoxyphenyl)-cyclobutyl]methyl]-β,N-dimethyl-m-dithian-2-propanamin-amidosulfat (1:1) erhalten.

| $C_{30}H_{43}NO_4S_2 \cdot H_3NO_3S$: | | | |
|---|---|---|---|
| Ber.: | C 53.80, | H 7.02, | N 4.65 % |
| Gef.: | C 53.57, | H 7.38, | N 4.16 %. |

Beispiel 26

(A) In analoger Weise wie in Beispiel 1, Absatz (C) beschrieben, wurde aus 1-(3,4-Dimethoxyphenyl)-N-methylcyclobutanmethylamin und 1-Brom-2-methyl-3-chlorpropan das rac-N-(3-Chlor-2-methylpropyl)-1-(3,4-dimethoxyphenyl)-N-methylcyclobutanmethanamin als ein dickflüssiges Oel (Sdp. 150°/260 Pa) erhalten, welches direkt in die nächste Stufe eingesetzt wurde.

(B) In analoger Weise wie in Beispiel 1, Absatz (D) beschrieben, wurde aus 2-(3,4,5-Trimethoxyphenyl)-m-dithian und rac-N-(3-Chlor-2-methylpropyl)-1-(3,4-dimethoxyphenyl)-N-methylcyclobutanmethanamin das rac-N-[[1-(3,4-Dimethoxyphenyl)cyclobutyl]methyl]-β,N-dimethyl-2-(3,4,5-trimethoxyphenyl)-m-dithian-2-propanamin als dickflüssiges Oel erhalten.

| $C_{31}H_{45}NO_5S_2$: | | | |
|---|---|---|---|
| Ber.: | C 64.67, | H 7.88, | N 2.43 % |
| Gef.: | C 64.65, | H 8.19, | N 2.35 %. |

Beispiel 27

In analoger Weise wie in Beispiel 1, Absatz (D) beschrieben, wurde aus 2-(4-Chlorphenyl)-m-dithian und rac-N-(3-Chlor-2-methylpropyl)-1-(3,4-dimethoxyphenyl)-N-methylcyclobutanmethanamin das rac-2-(4-Chlorphenyl)-N-[[1-(3,4-dimethoxyphenyl)cyclobutyl]methyl]-β,N-dimethyl-m-dithian-2-propanamin als dickflüssiges Oel erhalten.

| $C_{28}H_{38}ClNO_2S_2$: | | | |
|---|---|---|---|
| Ber.: | C 64.65, | H 7.36, | N 2.69 % |
| Gef.: | C 65.22, | H 7.54, | N 2.74 %. |

Beispiel 28

In analoger Weise wie in Beispiel 1, Absatz (D) beschrieben, wurde aus 2-(3,4-Dimethoxyphenyl)-m-dithian und rac-N-(3-Chlor-2-methylpropyl)-1-(3,4-dimethoxyphenyl)-N-methylcyclobutanmethanamin das rac-2-(3,4-Dimethoxyphenyl)-N-[[1-(3,4-dimethoxyphenyl)cyclobutyl]methyl]-β,N-dimethyl-m-dithian-2-propanamin als dickflüssiges Oel erhalten.

| $C_{30}H_{43}NO_4S_2$: | | | |
|---|---|---|---|
| Ber.: | C 66.02, | H 7.94, | N 2.57 % |
| Gef.: | C 65.99, | H 8.03, | N 2.43 %. |

Beispiel 29

(A) In analoger Weise wie in Beispiel 1, Absatz (A) beschrieben, wurde aus 1-(4-Chlorphenyl)-cyclobutancarbonitril [hergestellt aus 4-Chlorphenylacetonitril nach J. Org. Chem. 36, 1308 (1971)] das 1-(4-Chlorphenyl)cyclobutanmethanamin als ein farbloses Oel (Sdp. 125°/650 Pa) erhalten, welches direkt in die nächste Stufe eingesetzt wurde.

(B) In analoger Weise wie in Beispiel 1, Absatz (B) beschrieben, wurde aus 1-(4-Chlorphenyl)-cyclobutanmethanamin das 1-(Chlorphenyl)-N-methylcyclobutanmethanamin als ein farbloses Oel (Sdp. 108°/260 Pa) erhalten, welches direkt in die nächste Stufe eingesetzt wurde.

(C) In analoger Weise wie in Beispiel 1, Absatz (C) beschrieben, wurde aus 1-(4-Chlorphenyl)-N-methylcyclobutanmethanamin und 1-Brom-3-chlorpropan das N-(3-Chlorpropyl)-1-(4-chlorphenyl)-cyclobutanmethanamin als dickflüssiges Oel erhalten, welches direkt in die nächste Stufe eingesetzt wurde.

(D) In analoger Weise wie in Beispiel 1, Absatz (D) beschrieben, wurde aus 2-(3,4,5-Trimethoxyphenyl)-m-dithian und N-(3-Chlorpropyl)-1-(4-chlorphenyl)cyclobutanmethanamin das N-[[1-(4-Chlorphenyl)-cyclobutyl]methyl]-N-methyl-2-(3,4,5-trimethoxyphenyl)-m-dithian-2-propanamin-hydrochlorid als Festkörper erhalten.

| $C_{28}H_{38}ClNO_3S_2 \cdot HCl$: | | | |
|---|---|---|---|
| Ber.: | C 58.73, | H 6.86, | N 2.45 % |
| Gef.: | C 58.02, | H 7.29, | N 2.27 %. |

Beispiel 30

In analoger Weise wie in Beispiel 1, Absatz (D) beschrieben, wurde aus 2-(4-Chlorphenyl)-m-dithian und N-(3-Chlorpropyl-1-(4-chlorphenyl)cyclobutanmethanamin das 2-(4-Chlorphenyl)-N-[[1-(4-chlorphenyl)-cyclobutyl)methyl]-N-methyl-m-dithian-2-propanamin als dickflüssiges Oel erhalten.

| $C_{25}H_{31}Cl_2NS_2$: | | | |
|---|---|---|---|
| Ber.: | C 62.48, | H 6.50, | N 2.91 % |
| Gef.: | C 62.69, | H 6.63, | N 2.82 %. |

Beispiel 31

In analoger Weise wie in Beispiel 1, Absatz (D) beschrieben, wurde aus 2-(3,4-Dimethoxyphenyl)-m-dithian und N-(3-Chlorpropyl-1-(4-chlorphenyl)cyclobutanmethanamin das N-[(1-(4-Chlorphenyl)cyclobutyl]-methyl]-2-(3,4-dimethoxyphenyl)-N-methyl-m-dithian-2-propanamin als dickflüssiges Oel erhalten.

| $C_{27}H_{36}ClNO_2S_2$: | | | |
|---|---|---|---|
| Ber.: | C 64.07, H | 7.17, | N 2.77 % |
| Gef.: | C 63.97, H | 7.37, | N 2.64 %. |

Beispiel 32

(A) In analoger Weise wie in Beispiel 1, Absatz (C) beschrieben, wurde aus 1-(4-Chlorphenyl)-N-methylcyclobutanmethanamin und 1-Brom-2-methyl-3-chlorpropan das rac-N-(3-Chlor-2-methylpropyl)-1-(4-chlorphenyl)-N-methylcyclobutanmethanamin als dickflüssiges Oel erhalten, welches direkt in die nächste Stufe eingesetzt wurde.
(B) In analoger Weise wie in Beispiel 1, Absatz (D) beschrieben, wurde aus 2-(3,4-Dimethoxyphenyl)-m-dithian und rac-N-(3-Chlor-2-methylpropyl)-1-(4-chlorphenyl)-N-methylcyclobutanmethanamin das rac-N-[[1-(4-Chlorphenyl)cyclobutyl]methyl]-2-(3,4-dimethoxyphenyl)-β,N-dimethyl-m-dithian-2-propanamin-hydrochlorid als Festkörper erhalten.

| $C_{28}H_{38}ClNO_2S_2 \cdot HCl$: | | | |
|---|---|---|---|
| Ber.: | C 60.42, | H 7.06, | N 2.52 % |
| Gef.: | C 60.64, | H 7.39, | N 2.42 %. |

Beispiel 33

In analoger Weise wie in Beispiel 1, Absatz (D) beschrieben, wurde aus 2-(3,4-Ethylendioxyphenyl)-m-dithian und rac-N-(3-Chlor-2-methylpropyl)-1-(4-chlorphenyl)-N-methylcyclobutanmethanamin das rac-2-(1,4-Benzodioxan-6-yl)-N-[[1-(4-chlorphenyl)cyclobutyl]methyl]-β,N-dimethyl-m-dithian-2-propanamin als dickflüssiges Oel erhalten.

| $C_{28}H_{36}ClNO_2S_2$: | | | |
|---|---|---|---|
| Ber.: | C 64.90, | H 7.00, | N 2.70 % |
| Gef.: | C 65.12, | H 7.17, | N 2.80 %. |

Beispiel 34

In analoger Weise wie in Beispiel 1, Absatz (D) beschrieben, wurde aus 2-(4-Chlorphenyl)-m-dithian und rac-N-(3-Chlor-2-methylpropyl)-1-(4-chlorphenyl)-N-methylcyclobutanmethanamin das rac-2-(4-Chlorphenyl)-N-[[1-(4-chlorphenyl)cyclobuty]methyl]-β,N-dimethyl-m-dithian-2-propanaminhydrochlorid als kristalliner Festkörper erhalten, Smp. 135-136° (aus Essigester).

| $C_{26}H_{33}Cl_2NS_2$: | | | |
|---|---|---|---|
| Ber.: | C 58.81, | H 6.45, | N 2.64 % |
| Gef.: | C 58.49, | H 6.61, | N 2.56 %. |

Beispiel A

Tabletten

Zusammensetzung:

| | |
|---|---|
| 1) rac-2-(4-Chlorphenyl)-N-[[1-(4-chlorphenyl)cyclobutyl]methyl]-β,N-dimethyl-m--dithian-2-propanamin hydrochlorid | 275 mg |
| 2) Milchzucker pulv. | 135 mg |
| 3) Maisstärke weiss | 55 mg |
| 4) Povidone K | 15 mg |
| 5) Maisstärke weiss | 15 mg |
| 6) Talk | 3 mg |
| 7) Magnesiumstearat | 2 mg |
| | Tablettengewicht 500 mg |

Herstellungsverfahren

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel B

Kapseln

Zusammensetzung:

| | |
|---|---|
| 1) rac-2-(4-Chlorphenyl)-N-[[1-(4-chlorphenyl)cyclobutyl]methyl]-β,N-dimethyl-m--dithian-2-propanamin hydrochlorid | 370 mg |
| 2) Milchzucker krist. | 100 mg |
| 3) Maisstärke weiss | 20 mg |
| 4) Talk | 9 mg |
| 5) Magnesiumstearat | 1 mg |
| | Kapselfüllgewicht 500 mg |

Der Wirkstoff wird mit dem Milchzucker intensiv gemischt. Dieser Mischung wird danach die Maisstärke, der Talk und das Magnesiumstearat zugemischt, und das Gemisch in Kapseln geeigneter Grösse abgefüllt.

Beispiel C

Injektionslösung

| | |
|---|---|
| 1) N-[[1-(3,4-Dimethoxyphenyl)cyclohexyl]methyl]-2-(3,4-dimethoxyphenyl)-N-methyl-m-dithian-2-propanaminhydrochlorid | 40 mg |
| 2) Natriumchlorid krist. puriss. | 42,5 mg |
| 3) Wasser zu Injektionszwecken | ad 5 ml |

Beispiel D

Infusionsflasche

3 mg Vincristinsulfat und 300 mg N-[[1-(3,4-Dimethoxyphenyl)cyclohexyl]methyl]-2-(3,4-dimethoxyphenyl)-N-methyl-m-dithian-2-propanaminhydrochlorid werden in 250 ml physiologischer Kochsalzlösung gelöst, sterilisiert und unter sterilen Bedingungen in eine Infusionsflasche gefüllt.

Beispiel E

Tabletten

Zusammensetzung:

| | |
|---|---|
| 1) (R)-(-)-N-(3,4-Dimethoxyphenethyl)-2-(3,4-dimethoxyphenyl)-$\beta$,N-dimethyl-m-dithian-2-propanamin-amidosulfat | 200 mg |
| 2) Milchzucker pulv. | 110 mg |
| 3) Maisstärke weiss | 55 mg |
| 4) Povidone K | 15 mg |
| 5) Maisstärke weiss | 15 mg |
| 6) Talk | 3 mg |
| 7) Magnesiumstearat | 2 mg |
| | Tablettengewicht 400 mg |

Herstellungsverfahren

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel F

Tabletten

Zusammensetzung:

| | |
|---|---|
| 1) (S)-(+)-N-(3,4-Dimethoxyphenethyl)-2-(3,4-dimethoxyphenyl)-$\beta$,N-dimethyl-m-dithian-2-propanamin-(1:2)-ascorbat | 200 mg |
| 2) Milchzucker pulv. | 110 mg |
| 3) Maisstärke weiss | 55 mg |
| 4) Povidone K | 15 mg |
| 5) Maisstärke weiss | 15 mg |
| 6) Talk | 3 mg |
| 7) Magnesiumstearat | 2 mg |
| | Tablettengewicht 400 mg |

Herstellungsverfahren

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel G

Wenn man nach den in den Beispielen A-F beschriebenen Verfahren arbeitet, können aus den folgenden, ebenfalls bevorzugten Verbindungen und ihren pharmazeutisch verwendbaren Salzen Tabletten, Kapseln, Injektionslösungen bzw. Infusionsflaschen hergestellt werden:

rac-N-[[1-(3,4-Dimethoxyphenyl)cyclohexyl]methyl]-β,N-dimethyl-2-(3,4,5-trimethoxyphenyl)-m-dithian-2-propanamin,

2-(3,4-Dimethoxyphenyl)-N-[[1-(3,4-dimethoxyphenyl)cyclopentyl]methyl]-N-methyl-m-dithian-2-propanamin,

rac-N-(4-Chlorphenethyl)-2-(4-chlorphenyl)-β,N-dimethyl-m-dithian-2-propanamin,

rac-N-(3,4-Dimethoxyphenethyl)-β,N-dimethyl-2-(2-naphthyl)-m-dithian-2-propanamin,

(S)-(+)-2-(3,4-Dimethoxyphenyl)-N-[[1-(3,4-dimethoxyphenyl)cyclobutyl]methyl]-β,N-dimethyl-m-dithian-2-propanamin und

(R)-(-)-2-(3,4-Dimethoxyphenyl)-N-[[1-(3,4-dimethoxyphenyl)cyclobutyl]methyl]-β,N-dimethyl-m-dithian-2-propanamin.

## Patentansprüche

**1.** Verbindungen der allgemeinen Formel

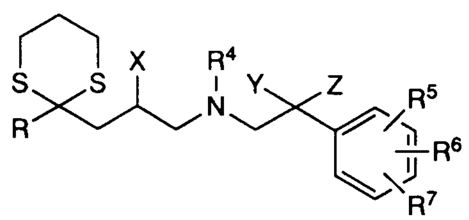

I

worin R einen Rest der Formel

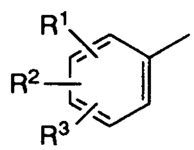

,

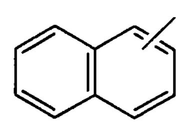

oder

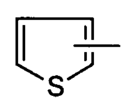

,

(a) (b) (c)

$R^1$, $R^2$ und $R^3$ je Wasserstoff, Halogen, niederes Alkyl, niederes Alkoxy, Aryl-niederes-Alkoxy, niederes Alkylthio, Trifluormethyl oder diniederes-Alkylamino oder zwei benachbarte dieser Reste zusammen Methylendioxy, Ethylendioxy, Trimethylen oder Tetramethylen; $R^4$ niederes Alkyl; $R^5$, $R^6$ und $R^7$ je Wasserstoff, Halogen, niederes Alkyl, niederes Alkoxy oder Aryl-niederes-Alkoxy oder zwei benachbarte dieser Reste zusammen Methylendioxy oder Ethylendioxy; X Wasserstoff oder niederes Alkyl; und Y und Z beide Wasserstoff oder niederes Alkyl oder zusammen Di-, Tri-, Tetra- oder Pentamethylen bedeuten; wobei aber X und Z nicht gleichzeitig Wasserstoff bedeuten und ausserdem rac-N-(3,4 Dimethoxyphenethyl)-2-(3,4-dimethoxyphenyl)-β,N-dimethyl-m-dithian-2-propylamin ausgeschlossen ist;

und Säureadditionssalze davon.

**2.** Verbindungen gemäss Anspruch 1, worin $R^4$ Methyl bedeutet.

**3.** Verbindungen gemäss Anspruch 1 oder 2, worin X Methyl bedeutet und das Kohlenstoffatom, an welchem dieses Methyl sitzt, die (R,S)-, (R)- oder (S)-Konfiguration aufweist.

**4.** Verbindungen gemäss Anspruch 3, worin die (R)- oder die (S)-Konfiguration vorliegt.

5. Verbindungen gemäss einem der Ansprüche 1-4, worin R einen Rest der Formel (a) oder (b) bedeutet.

6. Verbindungen gemäss Anspruch 5, worin R einen Rest der Formel (a) bedeutet und einer der Reste $R^1$, $R^2$ und $R^3$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^1$, $R^2$ und $R^3$ Wasserstoff und der dritte Halogen, insbesondere Chlor, bedeuten.

7. Verbindungen gemäss einem der Ansprüche 1-6, worin einer der Reste $R^5$, $R^6$ und $R^7$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^5$, $R^6$ und $R^7$ Wasserstoff und der dritte Halogen, insbesondere Chlor, niederes Alkyl, insbesondere Methyl, oder niederes Alkoxy, insbesondere Methoxy, bedeuten.

8. Verbindungen gemäss einem der Ansprüche 1-7, worin Y und Z beide Wasserstoff oder zusammen Tri-, Tetra- oder Pentamethylen bedeuten.

9. N-[[1-(3,4-Dimethoxyphenyl)cyclohexyl]methyl]-2-(3,4-dimethoxyphenyl)-N-methyl-m-dithian-2-propanamin.

10. rac-N-[[1-(3,4-Dimethoxyphenyl)cyclohexyl]methyl]-β,N-dimethyl-2-(3,4,5-trimethoxyphenyl)-m-dithian-2-propanamin.

11. 2-(3,4-Dimethoxyphenyl)-N-[[1-(3,4-dimethoxyphenyl)cyclopentyl]methyl]-N-methyl-m-dithian-2-propanamin.

12. rac-2-(4-Chlorphenyl)-N-[[1-(4-chlorphenyl)cyclobutyl]methyl]-β,N-dimethyl-m-dithian-2-propanamin.

13. (S)-(+)-2-(3,4-Dimethoxyphenyl)-N-[[1-(3,4-dimethoxyphenyl)cyclobutyl]methyl]-β,N-dimethyl-m-dithian-2-propanamin.

14. (R)-(-)-2-(3,4-Dimethoxyphenyl)-N-[[1-(3,4-dimethoxyphenyl) cyclobutyl]methyl]-β,N-dimethyl-m-dithian-2-propanamin.

15. (R)-(-)-N-(3,4-Dimethoxyphenethyl)-2-(3,4-dimethoxyphenyl)-β,N-dimethyl-m-dithian-2-propanamin.

16. (S)-(+)-N-(3,4-Dimethoxyphenethyl)-2-(3,4-dimethoxyphenyl)-β,N-dimethyl-m-dithian-2-propanamin.

17. rac-N-(4-Chlorphenethyl)-2-(4-chlorphenyl)-β,N-dimethyl-m-dithian-2-propanamin.

18. rac-N-(3,4-Dimethoxyphenethyl)-β,N-dimethyl-2-(2-naphthyl)-m-dithian-2-propanamin.

19. Säureadditionssalze, insbesondere Hydrochloride, Methansulfonate, Amidosulfate, Ascorbate und Oxalate von Verbindungen gemäss einem der Ansprüche 9-18.

20. Verbindungen gemäss einem der Ansprüche 1-19 zur Anwendung als therapeutische Wirkstoffe.

21. Verbindungen gemäss einem der Ansprüche 1-19 zur Anwendung bei der Verminderung und/oder Eliminierung der Mehrfachresistenz gegenüber Cytostatika in der Behandlung von Tumoren.

22. Verbindungen gemäss einem der Ansprüche 1-19 zur Anwendung bei der Verminderung und/oder Eliminierung der Chloroquinresistenz in der Behandlung von Malaria.

23. Verfahren zur Herstellung von Verbindungen gemäss einem der Ansprüche 1-19, dadurch gekennzeichnet, dass man eine Verbindung der allgemeinen Formel

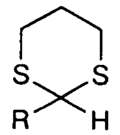

II

worin R die in Anspruch 1 angegebene Bedeutung hat, mit einer Verbindung der allgemeinen Formel

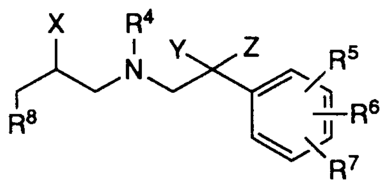

III

worin $R^8$ eine austretende Gruppe bedeutet und X, Y, Z,$R^4$, $R^5$, $R^6$ und $R^7$ die in Anspruch 1 angegebene Bedeutung haben,
umsetzt, erwünschtenfalls, eine erhaltene racemische Verbindung der Formel I in die entsprechenden Enantiomeren aufspaltet und/oder erwünschtenfalls eine erhaltene Verbindung der Formel I in ein pharmazeutisch verwendbares Säureadditionssalz überführt.

**24.** Arzneimittel, enthaltend eine Verbindung gemäss einem der Ansprüche 1-19 und ein therapeutisch inertes Excipiens.

**25.** Mittel enthaltend eine Verbindung gemäss einem der Ansprüche 1-19 und/oder rac-N-(3,4-Dimethoxyphenethyl)-2-(3,4-dimethoxyphenyl)-β,N-dimethyl-m-dithian-2-propanamin und/oder ein Säureadditionssalz davon und ein Cytostatikum als Kombinationspräparat zur gleichzeitigen getrennten, oder zeitlich abgestuften Anwendung in der Behandlung von malignen Tumoren und entsprechenden Metastasen.

**26.** Mittel zur Verminderung und/oder Eliminierung der Chloroquinresistenz in der Behandlung von Malaria enthaltend eine Verbindung gemäss einem der Ansprüche 1-19 und ein therapeutisch inertes Excipiens.

**27.** Verwendung einer Verbindung gemäss einem der Ansprüche 1-19 bei der Bekämpfung bzw. Verhütung von Krankheiten.

**28.** Verwendung einer Verbindung gemäss einem der Ansprüche 1-19 und/oder von rac-N-(3,4-Dimethoxyphenethyl)-2-(3,4-dimethoxyphenyl)-β,N-dimethyl-m-dithian-2-propanamin und von Säureadditionssalzen davon zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria.

**29.** Verwendung einer Verbindung gemäss einem der Ansprüche 1-19 und/oder von rac-N-(3,4-Dimethoxyphenethyl)-2-(3,4-dimethoxyphenyl)-β,N-dimethyl-m-dithian-2-propanamin und von Säureadditionssalzen davon zur Herstellung von Mitteln zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria.

**Patentansprüche für folgende Vertragsstaaten : GR, ES**

**1.** Verfahren zur Herstellung von Verbindungen der allgemeinen Formel

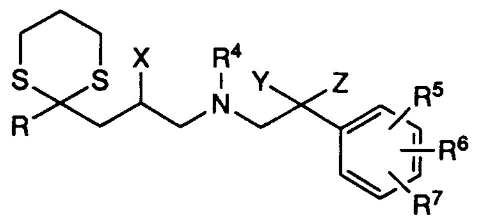

I

worin R einen Rest der Formel

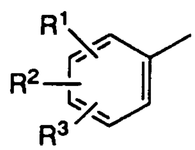 , 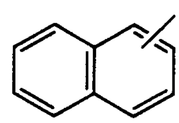 oder 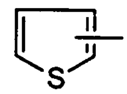 ,

(a) (b) (c)

$R^1$, $R^2$ und $R^3$ je Wasserstoff, Halogen, niederes Alkyl, niederes Alkoxy, Aryl-niederes-Alkoxy, niederes Alkylthio, Trifluormethyl oder diniederes-Alkylamino oder zwei benachbarte dieser Reste zusammen Methylendioxy, Ethylendioxy, Trimethylen oder Tetramethylen; $R^4$ niederes Alkyl; $R^5$, $R^6$ und $R^7$ je Wasserstoff, Halogen, niederes Alkyl, niederes Alkoxy oder Aryl-niederes-Alkoxy oder zwei benachbarte dieser Reste zusammen Methylendioxy oder Ethylendioxy; X Wasserstoff oder niederes Alkyl; und Y und Z beide Wasserstoff oder niederes Alkyl oder zusammen Di-, Tri-, Tetra- oder Pentamethylen bedeuten; wobei aber X und Z nicht gleichzeitig Wasserstoff bedeuten und ausserdem rac-N-(3,4 Dimethoxyphenethyl)-2-(3,4-dimethoxyphenyl)-$\beta$,N-dimethyl-m-dithian-2-propylamin ausgeschlossen ist;
und Säureadditionssalzen davon, dadurch gekennzeichnet, dass man eine Verbindung der allgemeinen Formel

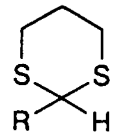

II

worin R die oben angegebene Bedeutung hat, mit einer Verbindung der allgemeinen Formel

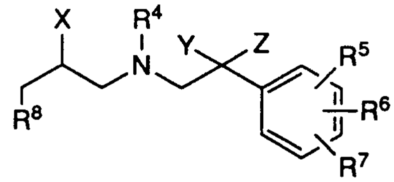

III

worin $R^8$ eine austretende Gruppe bedeutet und X, Y, Z,$R^4$, $R^5$, $R^6$ und $R^7$ die oben angegebene Bedeutung haben,
umsetzt, erwünschtenfalls, eine erhaltene racemische Verbindung der Formel I in die entsprechenden Enantiomeren aufspaltet und/oder erwünschtenfalls eine erhaltene Verbindung der Formel I in ein pharmazeutisch verwendbares Säureadditionssalz überführt.

2. Verfahren gemäss Anspruch 1, worin $R^4$ Methyl bedeutet.

3. Verfahren gemäss Anspruch 1 oder 2, worin X Methyl bedeutet und das Kohlenstoffatom, an welchem dieses Methyl sitzt, die (R,S)-, (R)-oder (S)-Konfiguration aufweist.

4. Verfahren gemäss Anspruch 3, worin die (R)- oder die (S)-Konfiguration vorliegt.

5. Verfahren gemäss einem der Ansprüche 1-4, worin R einen Rest der Formel (a) oder (b) bedeutet.

6. Verfahren gemäss Anspruch 5, worin R einen Rest der Formel (a) bedeutet und einer der Reste $R^1$, $R^2$ und $R^3$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^1$, $R^2$ und $R^3$ Wasserstoff und der dritte Halogen, insbesondere Chlor, bedeuten.

7. Verfahren gemäss einem der Ansprüche 1-6, worin einer der Reste $R^5$, $R^6$ und $R^7$ Wasserstoff und die beiden anderen je niederes Alkoxy, insbesondere Methoxy, oder - falls sie benachbart sind - zusammen Methylendioxy oder Ethylendioxy bedeuten oder zwei der Reste $R^5$, $R^6$ und $R^7$ Wasserstoff und der dritte Halogen, insbesondere Chlor, niederes Alkyl, insbesondere Methyl, oder niederes Alkoxy, insbesondere Methoxy, bedeuten.

8. Verfahren gemäss einem der Ansprüche 1-7, worin Y und Z beide Wasserstoff oder zusammen Tri-, Tetra- oder Pentamethylen bedeuten.

9. Verfahren gemäss Anspruch 1, dadurch gekennzeichnet, dass man N-[[1-(3,4-Dimethoxyphenyl)-cyclohexyl]methyl]-2-(3,4-dimethoxyphenyl)-N-methyl-m-dithian-2-propanamin herstellt.

10. Verfahren gemäss Anspruch 1, dadurch gekennzeichnet, dass man rac-N-[[1-(3,4-Dimethoxyphenyl)-cyclohexyl]methyl]-$\beta$,N-dimethyl-2-(3,4,5-trimethoxyphenyl)-m-dithian-2-propanamin herstellt.

11. Verfahren gemäss Anspruch 1, dadurch gekennzeichnet, dass man 2-(3,4-Dimethoxyphenyl)-N-[[1-(3,4-dimethoxyphenyl)cyclopentyl]methyl]-N-methyl-m-dithian-2-propanamin herstellt.

12. Verfahren gemäss Anspruch 1, dadurch gekennzeichnet, dass man rac-2-(4-Chlorphenyl)-N-[[1-(4-chlorphenyl)cyclobutyl]methyl]-$\beta$,N-dimethyl-m-dithian-2-propanamin herstellt.

13. Verfahren gemäss Anspruch 1, dadurch gekennzeichnet, dass man (S)-(+)-2-(3,4-Dimethoxyphenyl)-N-[[1-(3,4-dimethoxyphenyl)cyclobutyl]methyl]-$\beta$,N-dimethyl-m-dithian-2-propanamin herstellt.

14. Verfahren gemäss Anspruch 1, dadurch gekennzeichnet, dass man (R)-(-)-2-(3,4-Dimethoxyphenyl)-N-[-[1-(3,4-dimethoxyphenyl)cyclobutyl]methyl]-$\beta$,N-dimethyl-m-dithian-2-propanamin herstellt.

15. Verfahren gemäss Anspruch 1, dadurch gekennzeichnet, dass man (R)-(-)-N-(3,4-Dimethoxyphenethyl)-2-(3,4-dimethoxyphenyl)-$\beta$,N-dimethyl-m-dithian-2-propanamin herstellt.

16. Verfahren gemäss Anspruch 1, dadurch gekennzeichnet, dass man (S)-(+)-N-(3,4-Dimethoxyphenethyl)-2-(3,4-dimethoxyphenyl)-$\beta$,N-dimethyl-m-dithian-2-propanamin herstellt.

17. Verfahren gemäss Anspruch 1, dadurch gekennzeichnet, dass man rac-N-(4-Chlorphenethyl)-2-(4-chlorphenyl)-$\beta$,N-dimethyl-m-dithian-2-propanamin herstellt.

18. Verfahren gemäss Anspruch 1, dadurch gekennzeichnet, dass man rac-N-(3,4-Dimethoxyphenethyl)-$\beta$,N-dimethyl-2-(2-naphthyl)-m-dithian-2-propanamin herstellt.

19. Verfahren zur Herstellung eines Antitumormittels, dadurch gekennzeichnet, dass man eine Verbindung der Formel I in Anspruch 1 und/oder rac-N-(3,4-Dimethoxyphenethyl)-2-(3,4-dimethoxyphenyl)-$\beta$,N-dimethyl-m-dithian-2-propanamin und/oder ein Säureadditionssalz davon und ein Cytostatikum zusammen in eine galenische Darreichungsform bringt..

20. Verwendung einer Verbindung der Formel I in Anspruch 1 und/oder von rac-N-(3,4-Dimethoxyphenethyl)-2-(3,4-dimethoxyphenyl)-$\beta$,N-dimethyl-m-dithian-2-propanamin und/oder eines Säureadditionssalzes davon zur Herstellung von Mitteln zur Verminderung und/oder Eliminierung der Mehrfachresistenz gegenüber Cytostatika in der Behandlung von Tumoren oder zur Verminderung und/oder Eliminierung der Chloroquinresistenz in der Behandlung von Malaria.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 1406

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| D,X | US-A-4 003 914 (H. RAMUZ)<br><br>* Spalten 1-2; Spalten 11-12 *<br>--- | 1-3,5-8, 19,20, 23,24,27 | C07D339/08<br>C07D409/14<br>C07D409/12<br>C07D409/04<br>A61K31/385 |
| D,A | EUROPEAN JOURNAL OF MEDICINAL CHEMISTRY, Bd. 24, Nr. 5, September 1989, Seiten 493 - 496<br>H.RAMUZ ET AL. 'Ro 11-2933, a potential drug in the treatment of cancer and malaria: synthesis and physicochemical properties; potential metabolites'<br>* Seite 493 - Seite 496 *<br>----- | 1,20-22 | |

RECHERCHIERTE SACHGEBIETE (Int. Cl. 5)

C07D
A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 23 SEPTEMBER 1992 | RUSSELL F. ENGLISH |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

EP 92 11 1406

-C-

Bemerkung: Obwohl die Ansprüche 27,28 sich auf ein Verfahren zur Behandlung des menschlichen/tierischen Körpers beziehen (Art. 52(4) EPÜ), wurde die Recherche durchgeführt und gründete sich auf die angeführten Wirkungen der Verbindung.